(19) Europäisches Patentamt
European Patent Office
Office européen des brevets

(11) **EP 2 929 888 B1**

(12) **EUROPEAN PATENT SPECIFICATION**

(45) Date of publication and mention
of the grant of the patent:
**10.10.2018 Bulletin 2018/41**

(51) Int Cl.:
***A61K 36/076*** *(2006.01)*        ***A61P 37/08*** *(2006.01)*
***A61K 36/8884*** *(2006.01)*      ***A61K 36/27*** *(2006.01)*
***A61K 36/46*** *(2006.01)*

(21) Application number: **13861063.9**

(22) Date of filing: **18.10.2013**

(86) International application number:
**PCT/KR2013/009356**

(87) International publication number:
**WO 2014/088200 (12.06.2014 Gazette 2014/24)**

(54) **COMPOSITION COMPRISING PORIA COCOS WOLF BARK EXTRACT AS ACTIVE INGREDIENT FOR USE IN PREVENTING, IMPROVING, OR TREATING TH2-MEDIATED ALLERGIC DISEASES**

ZUSAMMENSETZUNG ZUR PRÄVENTION, LINDERUNG ODER BEHANDLUNG VON TH2-VERMITTELTEN ALLERGISCHEN ERKRANKUNGEN MIT TUCKAHOESCHALENEXTRAKT ALS WIRKSTOFF

COMPOSITION POUR PRÉVENIR, SOULAGER OU TRAITER DES MALADIES ALLERGIQUES À MÉDIATION PAR TH2 CONTENANT DE L'EXTRAIT DE PEAU DE TRUFFE DE VIRGINIE EN TANT QUE SUBSTANCE ACTIVE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **06.12.2012   KR 20120141325**
**06.12.2012   KR 20120141326**
**06.12.2012   KR 20120141328**
**01.02.2013   KR 20130012031**
**01.02.2013   KR 20130012015**
**01.02.2013   KR 20130012036**

(43) Date of publication of application:
**14.10.2015   Bulletin 2015/42**

(73) Proprietor: **Korea Food Research Institute Seongnam-si, Gyeonggi-do 463-746 (KR)**

(72) Inventors:
• **SHON, Dong-Hwa**
**Yongin-si**
**Gyeonggi-do 446-519 (KR)**
• **SHIN, Hee-Soon**
**Anyang-si**
**Gyeonggi-do 430-010 (KR)**
• **BAE, Min-Jung**
**Anyang-si**
**Gyeonggi-do 430-010 (KR)**

• **CHAI, Ok-Hee**
**Jeonju-si**
**Jeollabuk-do 561-785 (KR)**
• **KANG, Chang-Yuil**
**Seoul 137-851 (KR)**
• **CHOI, Dae-Woon**
**Taebaek-si**
**Gangwon-do 235-030 (KR)**
• **JUNG, Sun-Young**
**Seoul 138-819 (KR)**
• **CHOI, Gye-Young**
**Busan 609-310 (KR)**
• **RHO, Jeong-Hae**
**Seongnam-si**
**Gyeonggi-do 463-776 (KR)**
• **DO, Jeong-Ryong**
**Yongin-si**
**Gyeonggi-do 448-130 (KR)**

(74) Representative: **Plougmann Vingtoft a/s Rued Langgaards Vej 8 2300 Copenhagen S (DK)**

(56) References cited:
**EP-A1- 1 498 130        EP-A1- 2 305 268**
**WO-A1-2012/146194     KR-A- 20110 044 946**
**KR-A- 20120 010 026     KR-B1- 100 613 625**

- JUN HO LEE ET AL: "Rubiae Radix suppresses the activation of mast cells through the inhibition of Syk kinase for anti-allergic activity", JOURNAL OF PHARMACY AND PHARMACOLOGY, vol. 58, no. 4, 1 April 2006 (2006-04-01), pages 503-512, XP55216942, LONDON; GB ISSN: 0022-3573, DOI: 10.1211/jpp.58.4.0010
- GAO LI-JUN ET AL: "Studies on immunoregulation of polysaccharides-la from Radix Cynanchi Bungei", ZHONGGUO ZHONGYAO ZAZHI - CHINA JOURNAL OF CHINESE MATERIA MEDICA, ZHOGGUO YAOXUEHUI, BEIJING, CN, vol. 30, no. 17, 1 September 2005 (2005-09-01), pages 1352-1355, XP009186329, ISSN: 1001-5302
- DATABASE WPI Week 201219 Thomson Scientific, London, GB; AN 2011-Q63466 XP002745183, & CN 102 240 386 A (UNIV AFFILIATED SHUGUANG HOSPITAL SHANGH) 16 November 2011 (2011-11-16)
- ROZEMA EVELIEN ET AL: "Effects on inflammatory responses by the sphingoid base 4,8-sphingadienine", INTERNATIONAL JOURNAL OF MOLECULAR MEDICINE, SPANDIDOS PUBLICATIONS, GR, vol. 30, no. 3, 1 September 2012 (2012-09-01), pages 703-707, XP009186331, ISSN: 1107-3756
- CHUNXIA C ET AL: "Extracts of Arisaema rhizomatum C.E.C. Fischer attenuate inflammatory response on collagen-induced arthritis in BALB/c mice", JOURNAL OF ETHNOPHARMACOLOGY, ELSEVIER IRELAND LTD, IE, vol. 133, no. 2, 27 January 2011 (2011-01-27), pages 573-582, XP027596139, ISSN: 0378-8741, DOI: 10.1016/J.JEP.2010.10.035 [retrieved on 2011-01-11]
- J. JEON ET AL.: 'Water extract of Cynanchi atrati Radix regulates inflammation and apoptotic cell death through suppression of IKK-mediated NF-K B signaling' JOURNAL OF ETHNOPHARMACOLOGY vol. 137, 2011, pages 626 - 634, XP028383242
- K. YASUKAWA ET AL.: '3beta-p-Hydroxybenzoyldehydrotumulosic acid from poria cocos, and its anti-inflammatory effect' PHYTOCHEMISTRY vol. 48, no. 8, 1998, pages 1357 - 1360, XP004289994
- DATABASE WPI Week 200839 Thomson Scientific, London, GB; AN 2008-G05093 & CN 101 095 928 A (GUAN Y) 2 January 2008 (2008-01-02)

**Description**

**FIELD OF THE INVENTION**

The present invention relates to a composition for use in preventing, improving Th2-mediated immune diseases comprising *Poria cocos* Wolf bark extract as active ingredient

**BACKGROUND OF THE INVENTION**

In recent years, allergies have increased due to various reason including changing dietary habits, better hygiene and intensified pollution along with advancement in income levels, and about 20-25% of the entire population have symptoms of, for example, atopic dermatitis (AD), asthma and rhinitis. These significantly affect the quality of life, and may lead to serious social issues. Also, it cannot be ignored that food allergies are found in 6-8% of infants and also cause 35% of children's ADs.

Until now, a medicine that shows definite effects against various allergies has not existed and, thus, treating children who have these allergies has been especially difficult even though children's ADs have been on the rise. Particularly, drugs that have similar characteristics as steroids bring about serious side effects so there are currently attempts around the world to prepare for alternative solutions. To combat these allergies, materials that are expected to have positive results without any toxicity or side effects must be extracted from natural substances and foods, which then should be actively utilized. Moreover, the anti-allergy mechanism of action for active materials and their components must be also examined in detail.

The main-stream medicine popular in the 21st century have been those for allergies and diabetes (Japan's Nikkei Sankyo Newspapers). In the United States, around 50 million people possess many different kinds of allergies (drug and medicine market, $5.2 billion annually). In Japan, there exists a market for functional foods worth around 60 billion JPY while that of drugs and medicine related to allergies is worth 156 billion JPY annually, compared to the Korean market for drugs and medicine, which is worth around 50 billion KRW. Looking at the recent trend of patents in functional foods, most of the ones submitted are in the area of "anti-obesity", followed by "anti-allergy", an area that is anticipated to be of much added value but also requires necessary preparation.

There has been an increasing need for development of health functional foods, emphasizing immune function controls or activation of allergy suppression, which has resulted in numerous patents submitted to the Korea Food and Drug Administration. Also, according to patent analyses in Japan during recent years, the future potential for functional foods was reported to lie in the activation of "anti-obesity" and then "anti-allergy".

The term "allergy" has been originated from a Greek word "allos", which means "hypersensitive reaction", and refers to the "deformed". In other words, an allergy is an occurrence caused by a substance, which has no effect on average people, inducing abnormally hypersensitive reaction such as hives, itching, nasal discharges and/or coughs on a particular individual due to malfunctioning of the immune system. In the last 20 years, the incidence rate of allergic diseases has been trending upward globally (1, 2). Primary reasons for this increase include more time spent indoors, development of new materials, sudden influx of allergy-inducing materials particularly from overseas, unstable immune statuses due to environmental contamination and stress, and changing dietary habits. Although the incidence rate of allergic diseases in the people of modern era has steadily increased, many only rely on well-known treatments such as anti-histamines or steroids without finding a fundamental treat for it (3).

Gell and Coombes (1963) traditionally divide allergic reactions into 4 categories based on time and types of expression (4). Types I through III are humoral immunologic responses (immediate-type) in which antibodies are involved while Type IV is a cellular immunologic response (delayed-type). Also, based on the types of cells and medium concerned with immune reactions, responses are divided into immediate or early responses in which symptoms are displayed within minutes, and late-stage responses in which symptoms appear after a few hours. For example, Type I takes the form of an immediate hypersensitive reaction while Type IV is a delayed hypersensitive reaction. Most allergic reactions are categorized as Type I, which includes diseases such as asthma, rhinitis, conjunctivitis, food and drug allergies and atopic dermatitis, and severe cases lead to anaphylaxis, which may risk one's life.

Type I immediate hypersensitive reactions are further divided into 2 steps. In the first step, allergen penetrates into a body and suppresses secretion of IgE and IgG1. Once the balance between Th1 cellular response, which produces IL-12 and IFN-r that increase secretion of IgG2a, and Th2 cellular response, which produces IL-4, IL-5 and IL-13, tips in the direction of Th2, IL-4, IL-13 and others are discharged due to an excessive hypersensitive reaction of Th2. Because of this effect, IgE specific antibodies produced by B cells are attached to mast cells or basophils, and, subsequently, an allergic crisis is ready, all of which is called the sensitization of allergens (5-7) (Refer to figure: "Allergic Diseases", "Th1/Th2 Balance").

The second step of allergic crisis is divided into early and late responses. In the early response, an allergen re-penetrates the body, stimulates mast cells and induces degranulative reactions, releasing histamines, lipid metabolites and Cytokine at this time to cause blood vessels to expand. In the late response, neutrophils, eosinophils, macrophage, Th2 cells and basophils infiltrate into a particular tissue and are activated, which leads to inflammation and symptoms of atopic dermatitis, rhinitis and asthma (7). Among these secreted matters of degranulation, histamine is the most well-known and used as an essential indicator for allergic symptoms due to its relations to immediate hypersensitive reactions (8).

On the other hand, food allergies have occurred in about 3 to 6% of children in developed countries for the past 10 years and show an upward trend (9). Food allergies, which are caused by food coming into a body through the digestive tract, belong primarily to Type I hypersensitive reaction. For allergy patients, food allergens penetrate through intestinal canals at an undigested state (4, 10) and, thus, the absorption of allergens inside intestines is the first step of an allergic crisis (11). Intestinal canals not only take care of digestion and absorption of nutrients but also are involved in various biomodulation. Primary characteristics of intestinal epithelial cells include digestion and absorption, the role as a barrier and transfer and conversion of food signals. As they also possess developed a special immune system (mucosal immunity) and nerve tissues, the role of digestive and intestinal canals must not be underestimated. Thus, as a barrier, intestinal epithelial cells restrict the penetration of large molecules by a tight junction; however, if the tight junction is damaged, the permeability increases due to the dysfunction of the barrier, which may result in diseases such as food allergies, celiac disease and acute pancreatitis (12-17). In other words, food allergies can be prevented by strengthening the barrier function of intestinal canals through stabilization of tight junction.

The research papers previously mentioned have reported, to a great extent, about the activation of the allergy inhibition in foods and natural products, which have been popularly used especially by Koreans in oriental medicine and home remedies to treat and prevent allergies (18, 19). These research mostly evaluate the inhibitory activity of degranulation in medicinal herbs such as areca nuts, Anemarrhena rhizome and fenugreek (20-33) and also report on Th1/Th2 immune response controls in polysaccharide components such as quercetin, Siberian gooseberries, garlic, perilla oil, konjac, lobulus and liriope rhizome in addition to those in Lingzhi mushrooms and Japanese soy sauce as well as royal jelly and cordyceps (34-44). Also reported were results on the activation which suppresses allergic inflammation in lactobacillus, fermented barley and cheonggukjang, a Korean fermented soybean soup (45-49).

EP1498130A1 discloses pharmaceutical compositions for treating immunological disorders including alcohol extracts of sclederma of *Poria cocos* Wolf, having antiinflammatory activity and regulating the secretion of cytokines. EP1498130A1 has no disclosure of *Poria cocos* Wolf bark extract.

EP2305268A1 discloses a Poria sclederma extract comprising a specified lanostane for treating an immune disorder such as an allergy like allergic asthma. EP2305268A1 has no disclosure of *Poria cocos* Wolf bark extract.

Jun Ho Lee et al. ("Rubiae Radix suppresses the activation of mast cells through the inhibitions of Syk kinase for anti-allergic acrivity", journal of pharmacy and pharmacology, vol. 58, no. 4, 1 April 2006, pages 503-512) discloses *Poria cocos* as natural products with inhibiting effects on antigen-induces degranulation in mast cells, but contains no disclosure of *Poria cocos* Wolf bark extract

## SUMMARY OF THE INVENTION

The present inventors have endeavored to develop natural products that may prevent or treat diseases and symptoms caused by Th1 or Th2 immune responses such as inflammatory diseases and allergies. As a result, the inventors have successfully completed this research by finding out that *Poria cocos* Wolf bark extract, *Cynanchym atratum* Bunge extract or *Arisaema amurense* extract inhibits production of Th1-related cytokines and Th2-related cytokines, penetration of allergens through intestinal epithelial cells, degranulation of mast cells; induces differentiation into Treg cells; and stabilizes Treg cells.

Accordingly, an object of the present invention is to provide a composition for use in preventing, improving, or treating Th2-mediated allergic diseases.

Other objects and advantages of the present invention will become apparent from the detailed description to follow taken in conjugation with the appended claims and drawings.

## BRIEF DESCRIPTION OF THE DRAWINGS

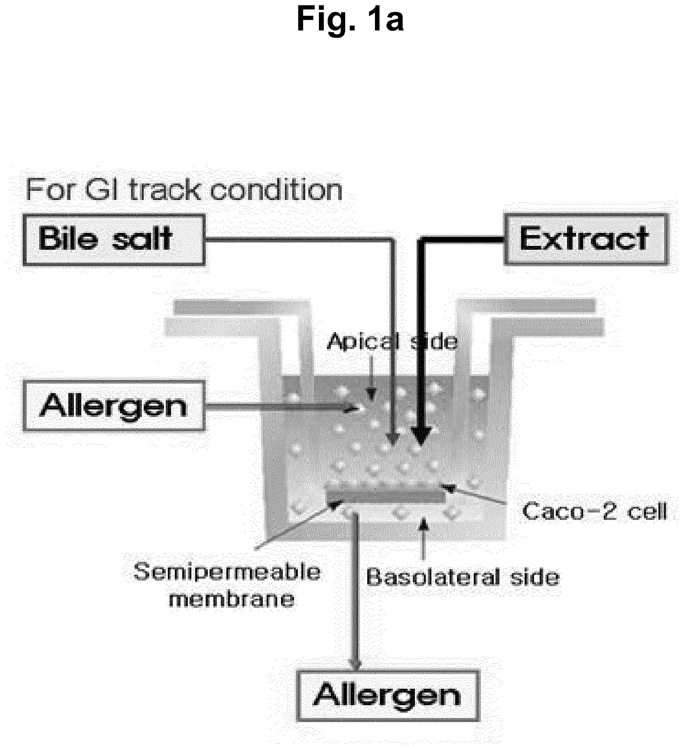

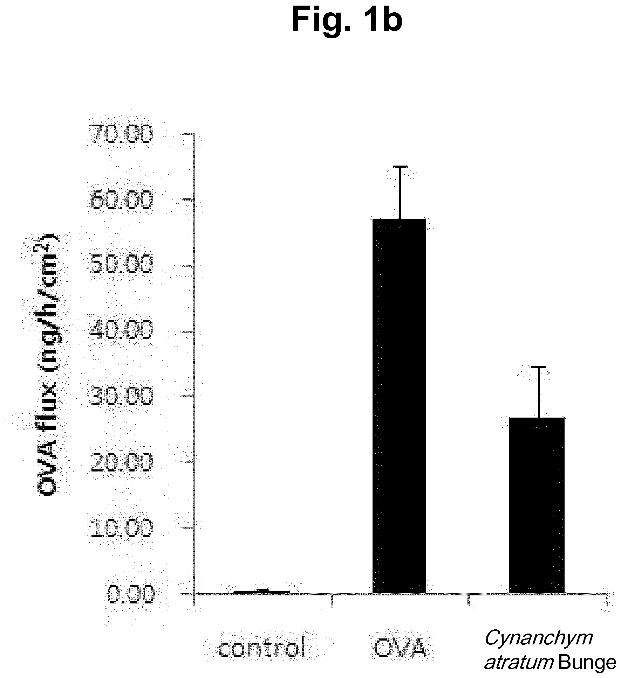

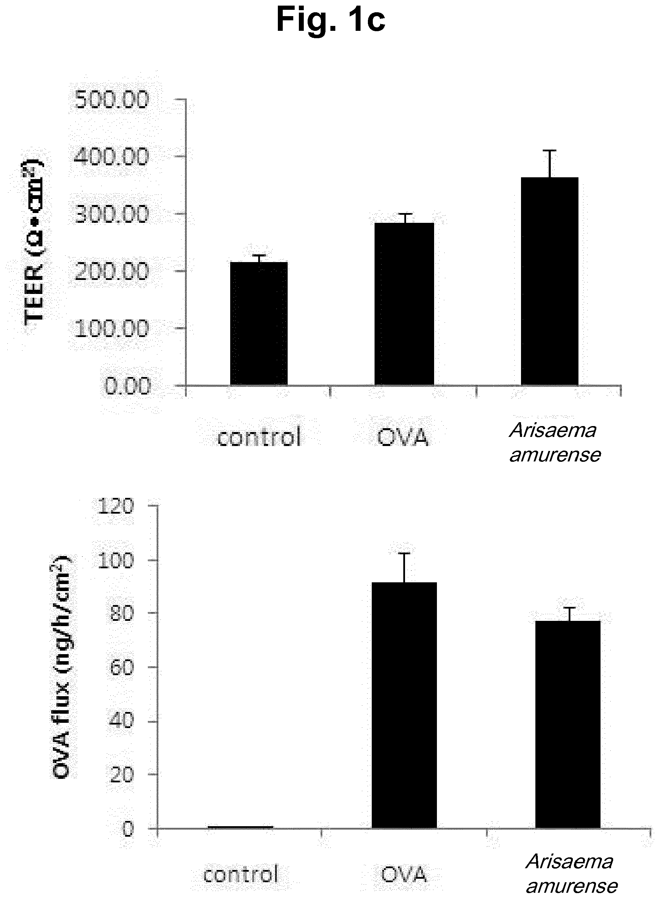

Fig. 1a shows a diagram of the test on penetration of allergens through intestinal epithelial cells. Fig. 1b shows the effect of *Cynanchym atratum* Bunge extract in penetration of allergens through epithelial cells, and fig. 1c shows the TEER (Transepitherial Electrical Resistance, $\Omega\times cm^2$) level and the effect of *Arisaema amurense* extract in penetration of allergens through epithelial cells.

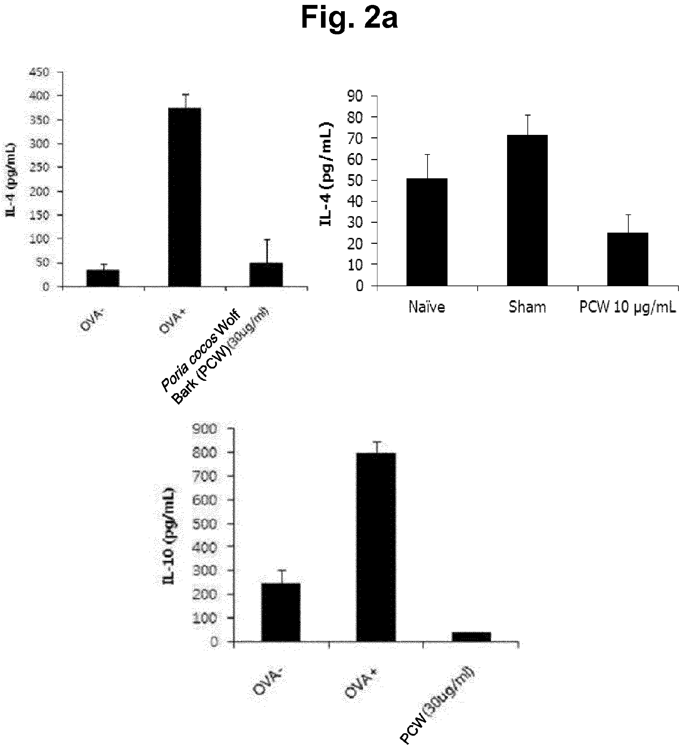

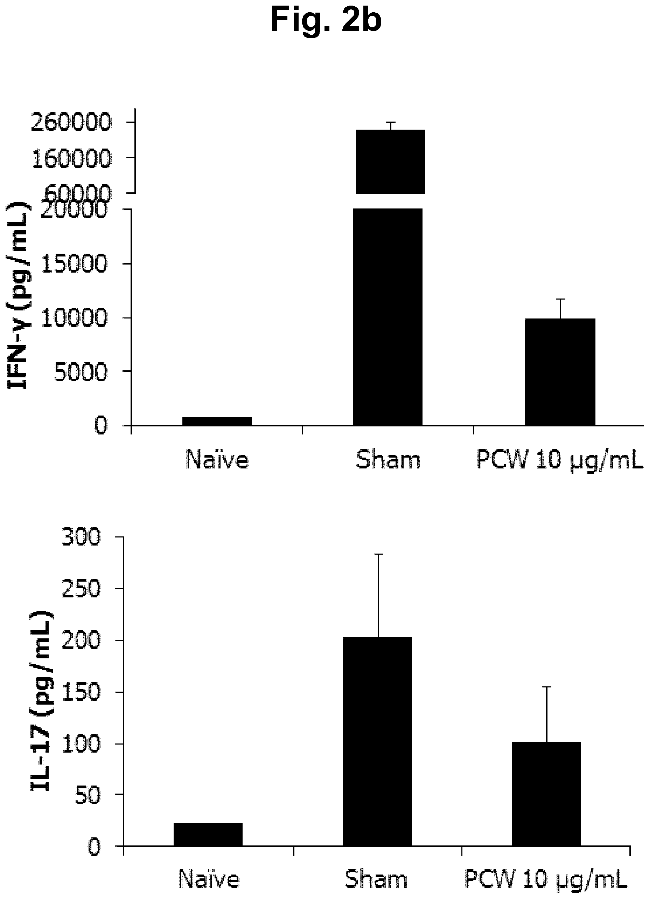

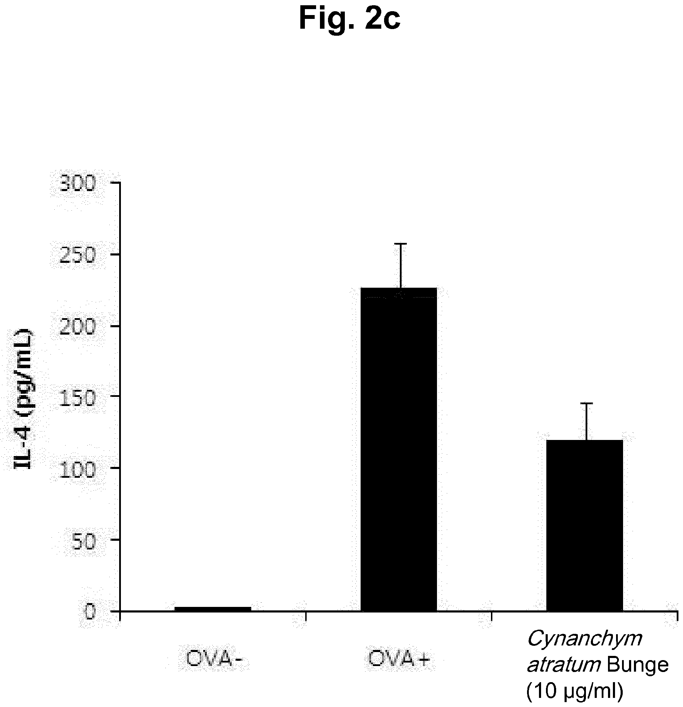

Fig. 2a shows the effect of *Poria cocos* Wolf bark extract in production of Th2-related cytokines (IL-4 and IL-10) of mouse splenocytes. Fig. 2b shows the effect of *Poria cocos* Wolf bark extract in production of Th-1 mediated cytokines (IFN-γ and IL-17) of mouse splenocytes. Fig. 2c shows the effect of *Arisaema amurense* extract in production of IL-4 of mouse splenocytes.

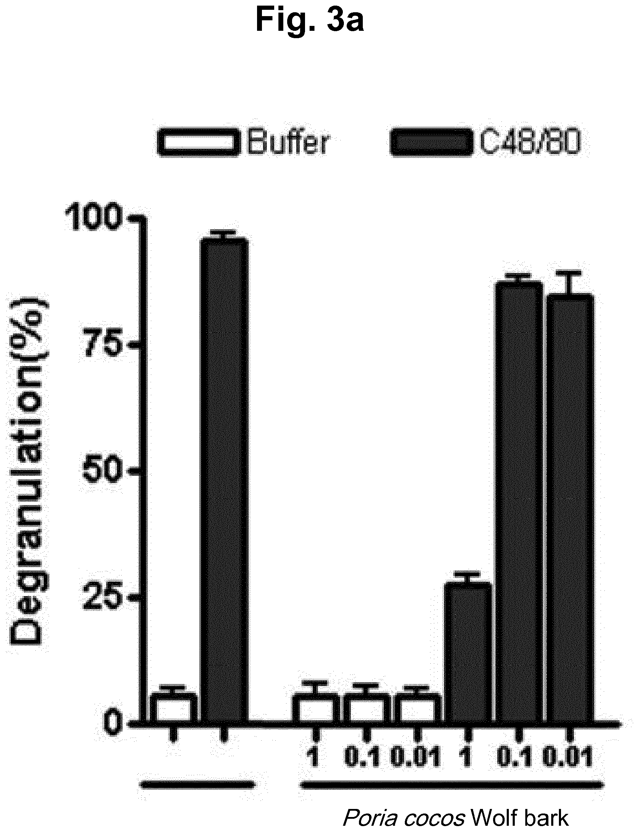

Figs. 3a-3c show the effects of *Poria cocos* Wolf bark extract, *Cynanchym atratum* Bunge extract and *Arisaema amurense* extract in degranulation of mouse mast cells, respectively.

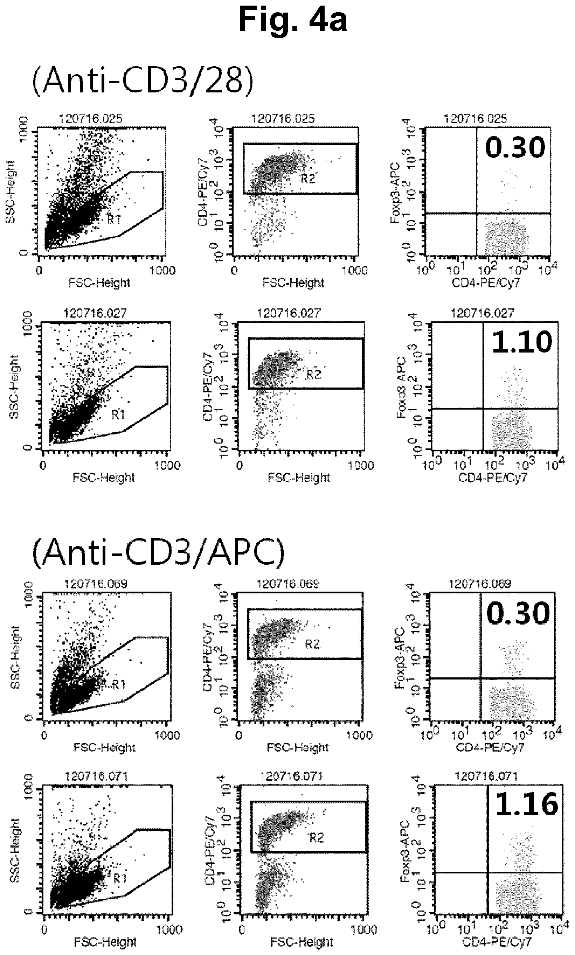

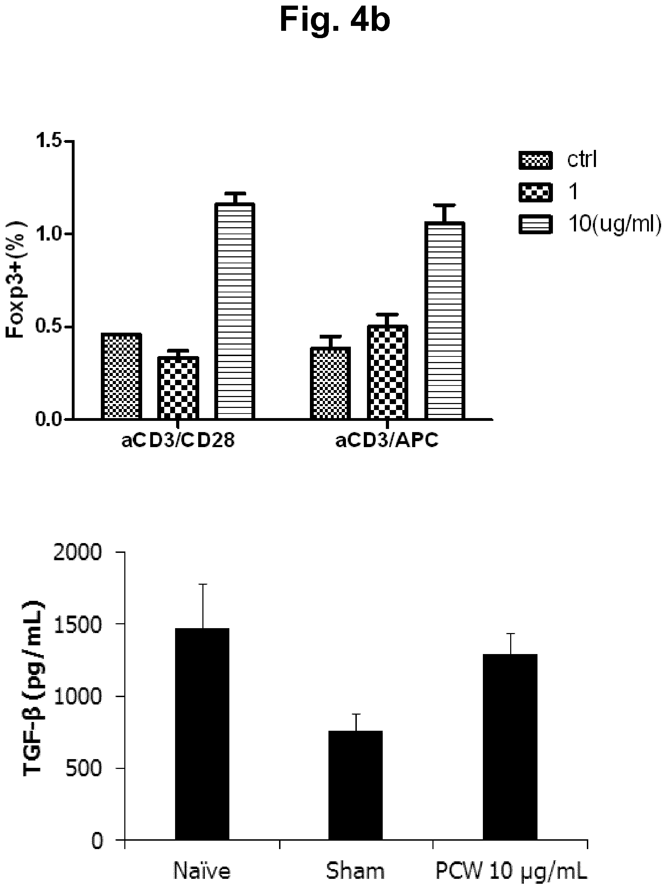

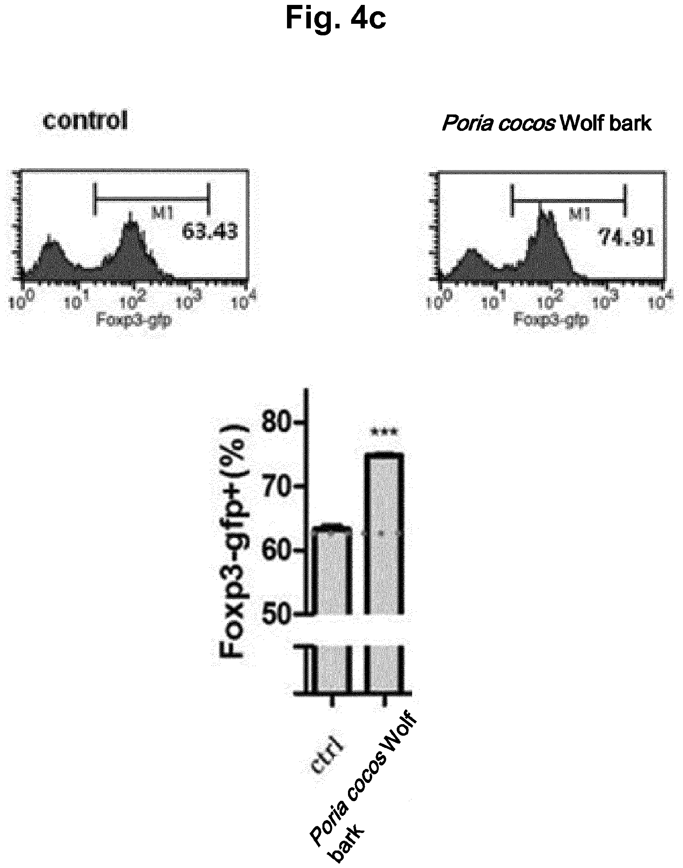

Figs. 4a and 4b show increase in Fox3p expression and TGF-β production as the effect of *Poria cocos* Wolf bark extract in induction of differentiation into Treg cells of mouse splenocytes and lymphatic glands, and fig. 4c shows the analysis result of stabilizing activity and graphs using High-Speed Cytometry Sorter as the effect of *Poria cocos* Wolf bark extract in stabilizing activity of Treg cells.

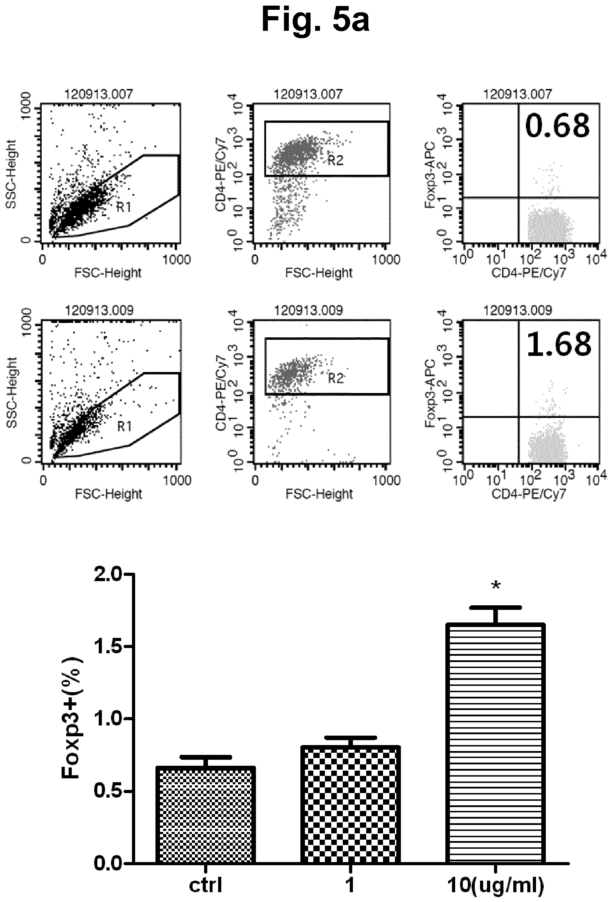

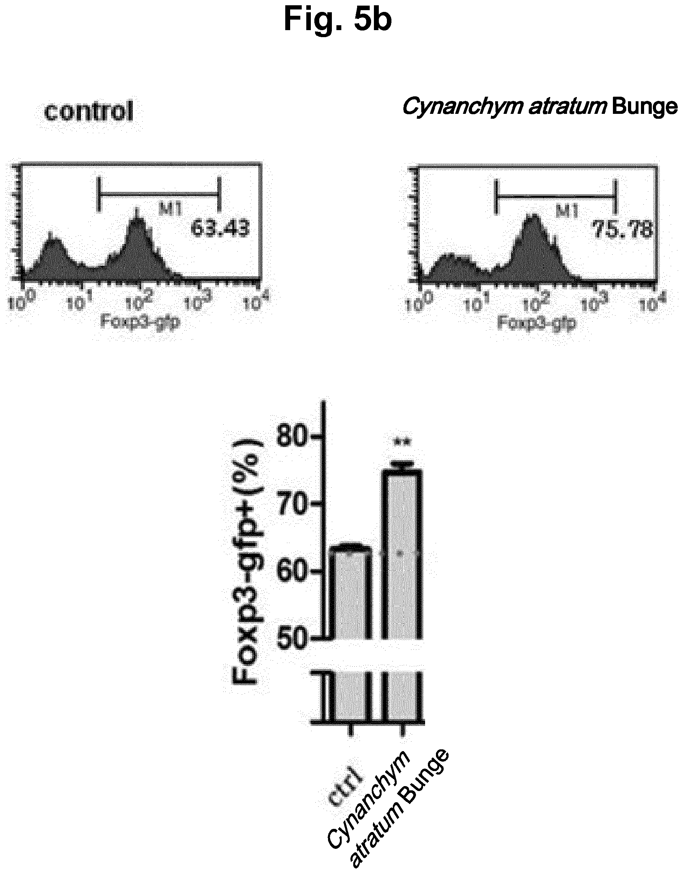

Fig. 5a shows the effect of *Cynanchym atratum* Bunge extract in induction of differentiation into Treg cells of mouse splenocytes and lymphatic glands, and fig. 5b shows the analysis result of stabilizing activity and graphs using High-Speed Cytometry Sorter as the effect of *Cynanchym atratum* Bunge extract in stabilizing activity of Treg cells.

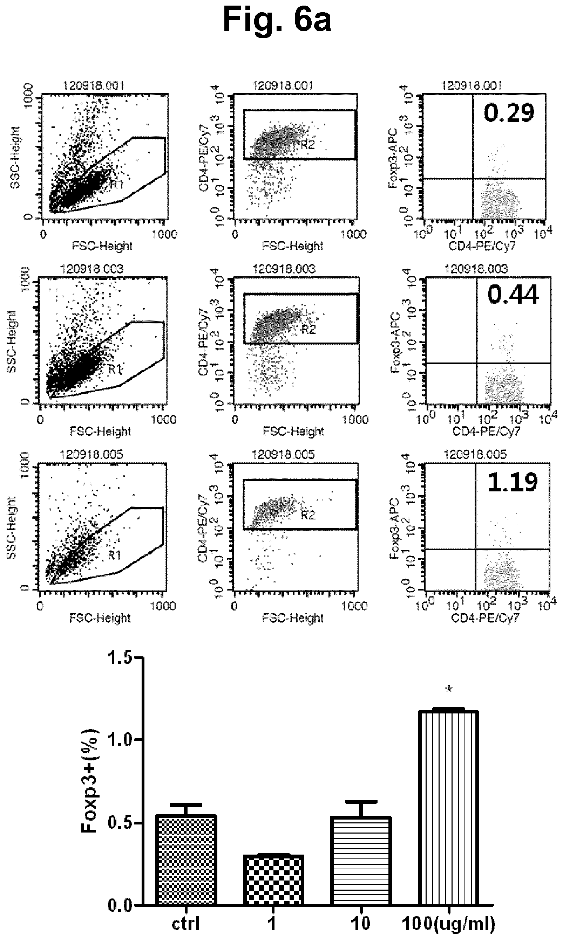

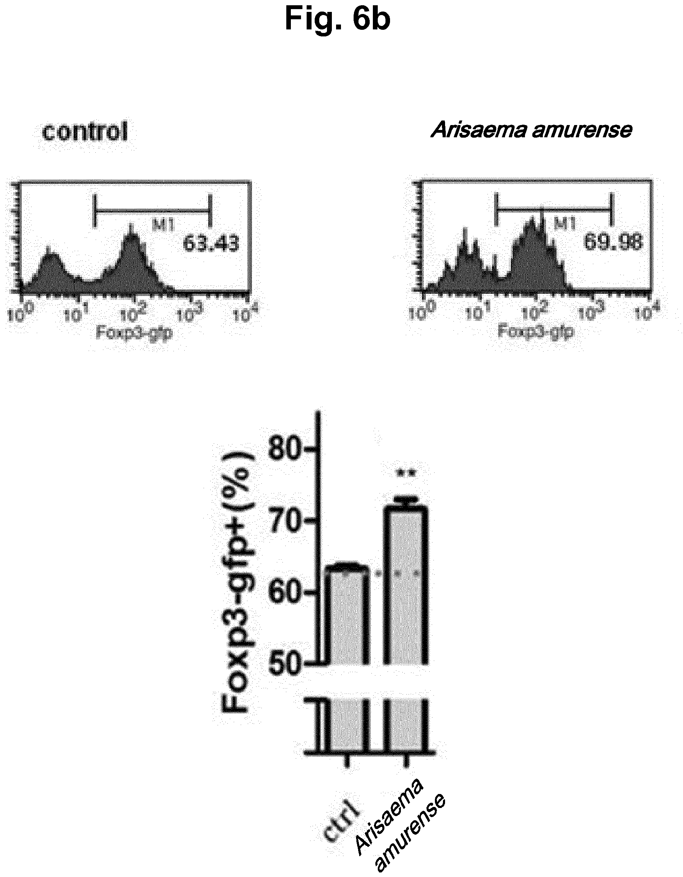

Fig. 6a shows the effect of *Arisaema amurense* extract in induction of differentiation into Treg cells of mouse splenocytes and lymphatic glands, and fig. 6b shows the analysis result of stabilizing activity and graphs using High-Speed Cytometry Sorter as the effect of *Arisaema amurense* extract in stabilizing activity of Treg cells.

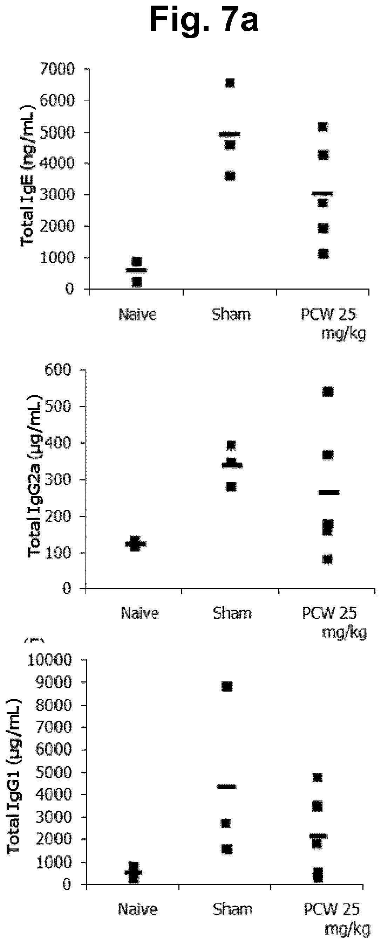

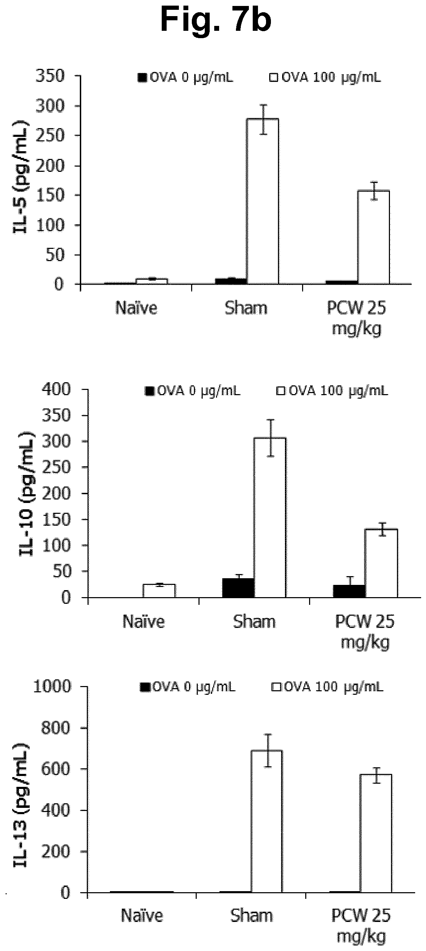

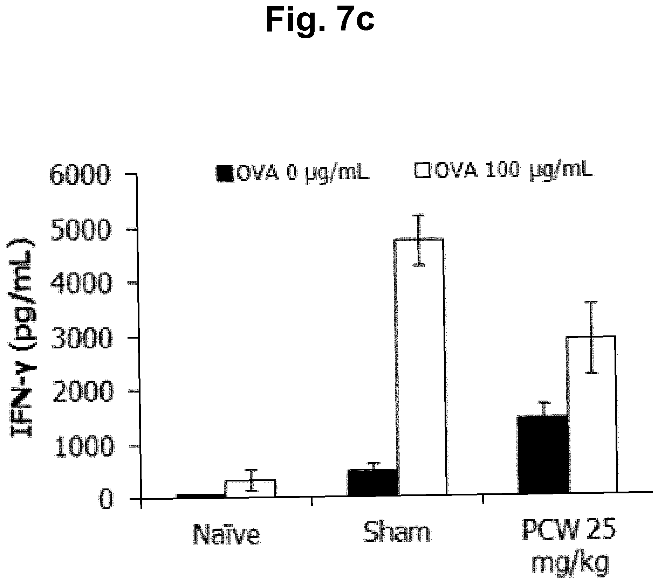

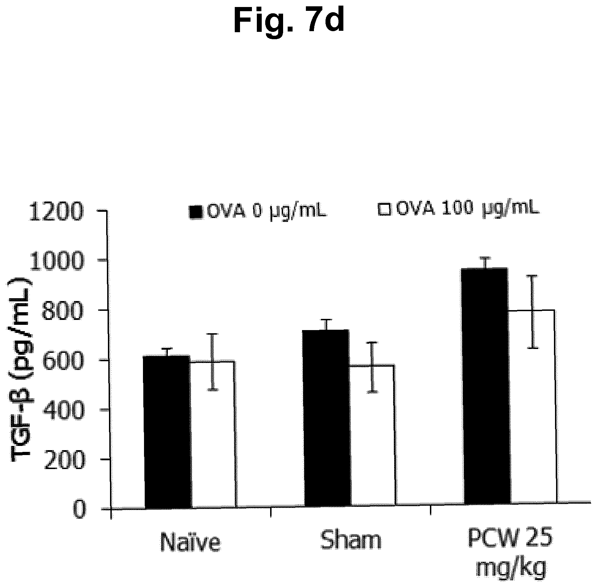

Fig. 7 represents anti-allergenic activity of *Poria cocos* Wolf bark extract *in vivo.* fig. 7a shows the inhibitory activity of *Poria cocos* Wolf bark extract in IgE, IgG2a and IgG1 of serum, fig. 7b shows the inhibitory activity of *Poria cocos* Wolf bark extract in Th-1 mediated cytokines production, fig. 7c shows the inhibitory activity in Th2-related cytokines production, fig. 7d shows the induction activity in differentiation into Treg cells.

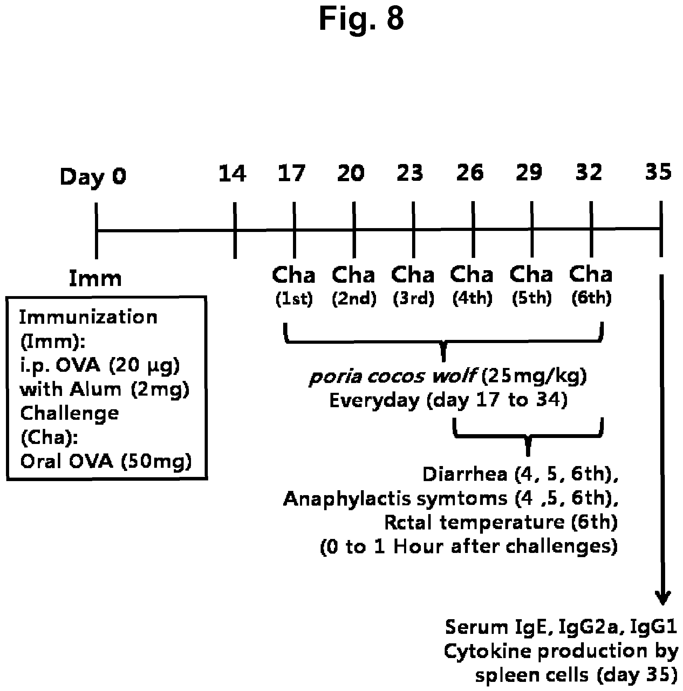

Fig. 8 represents schedule for the preparation of food allergy model.

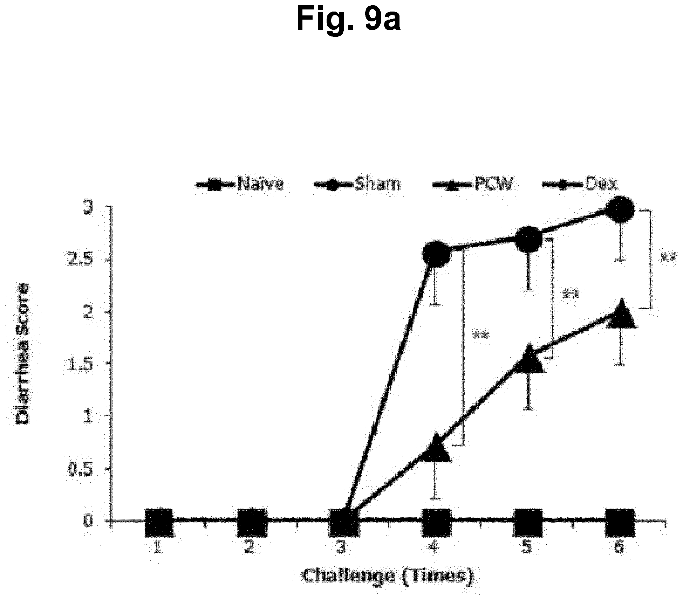

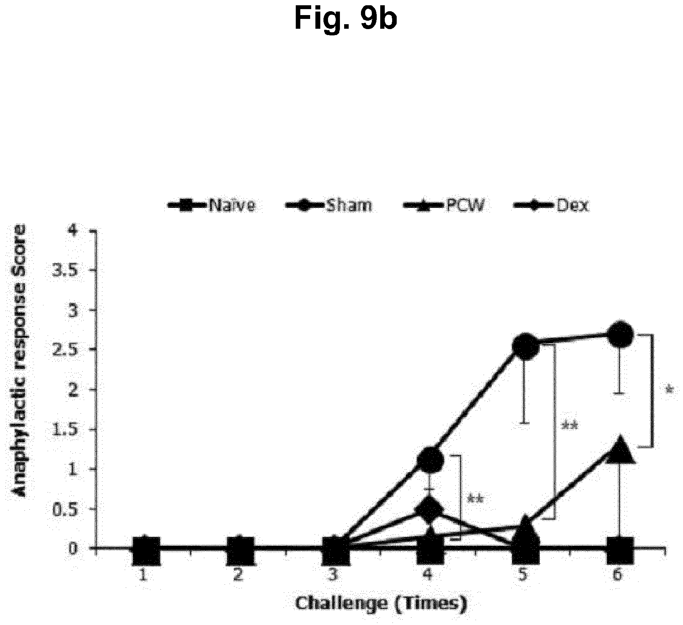

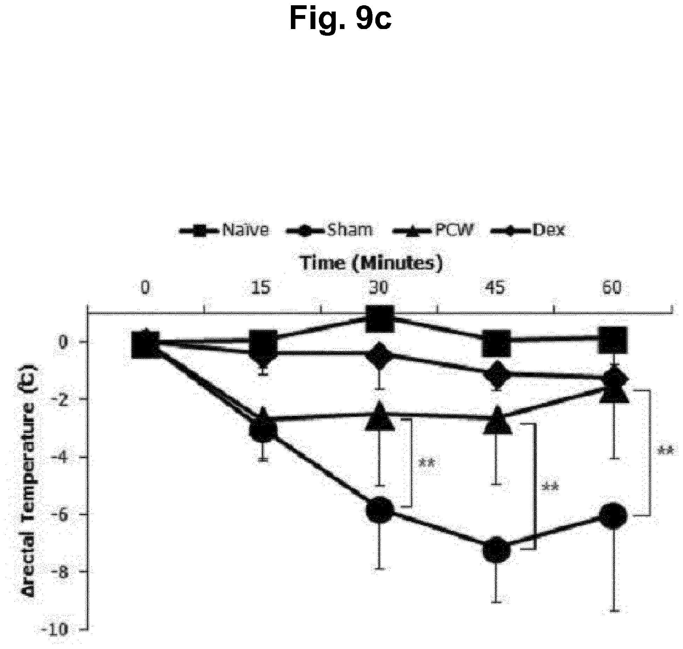

Fig. 9 represents the measurement result of *Poria cocos* Wolf bark extract effect in food allergy using anaphylaxis, the diarrhea response and the change of rectal temperature.

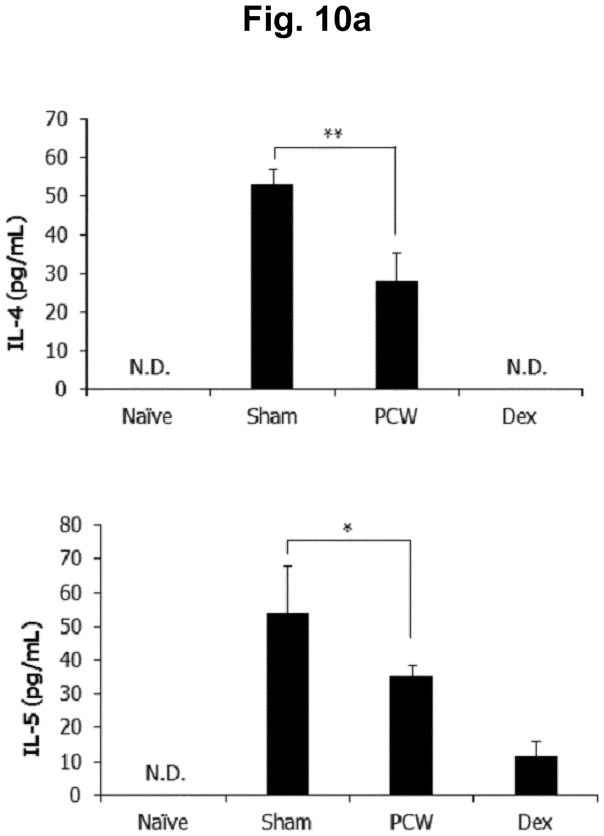

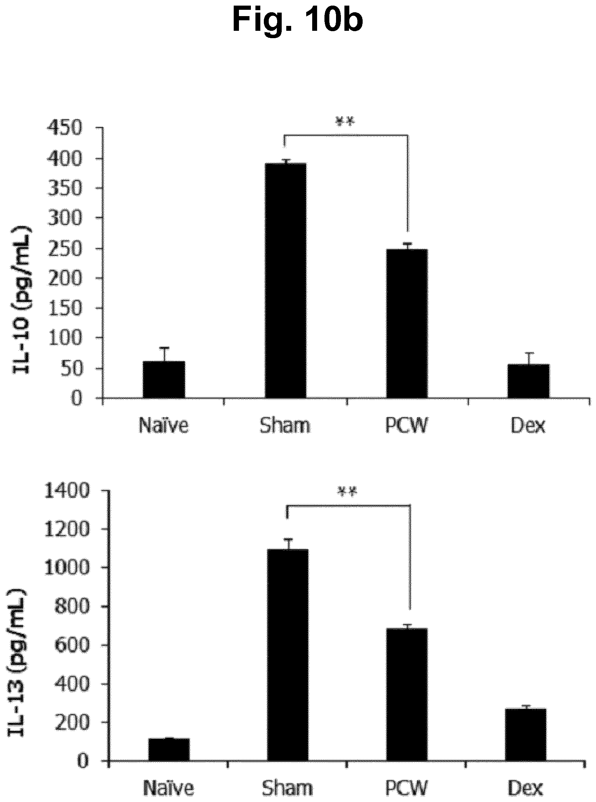

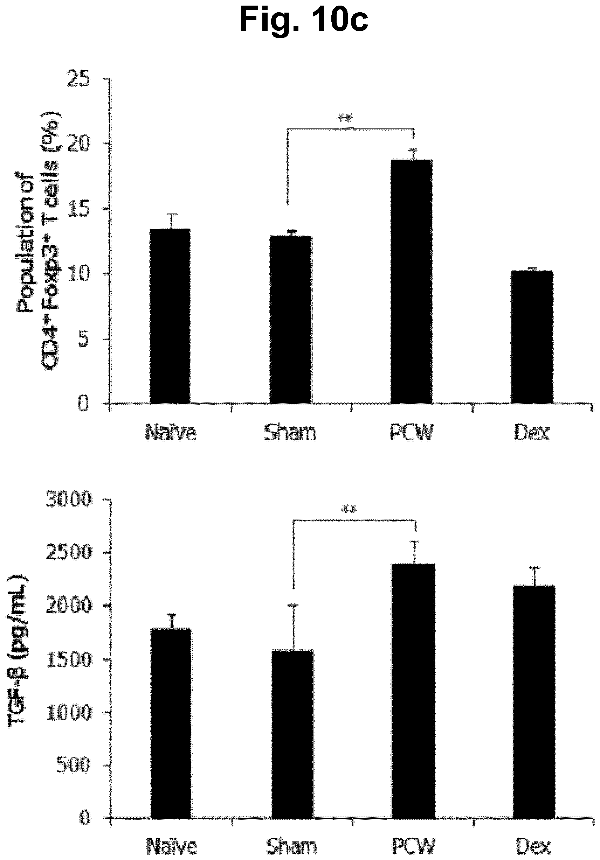

Fig. 10 shows the effect of *Poria cocos* Wolf bark extract in cytokine production of food allergy model splenocytes. Fig 10a shows the inhibitory activity of *Poria cocos* Wolf bark in Th2-related cytokines including IL-4, IL-5, IL-10 and IL-13 production, and fig. 10b shows the increase activity of *Poria cocos* Wolf bark in *in vivo* Treg cytokines including TGF-β production which is related to the induction of foxp3.

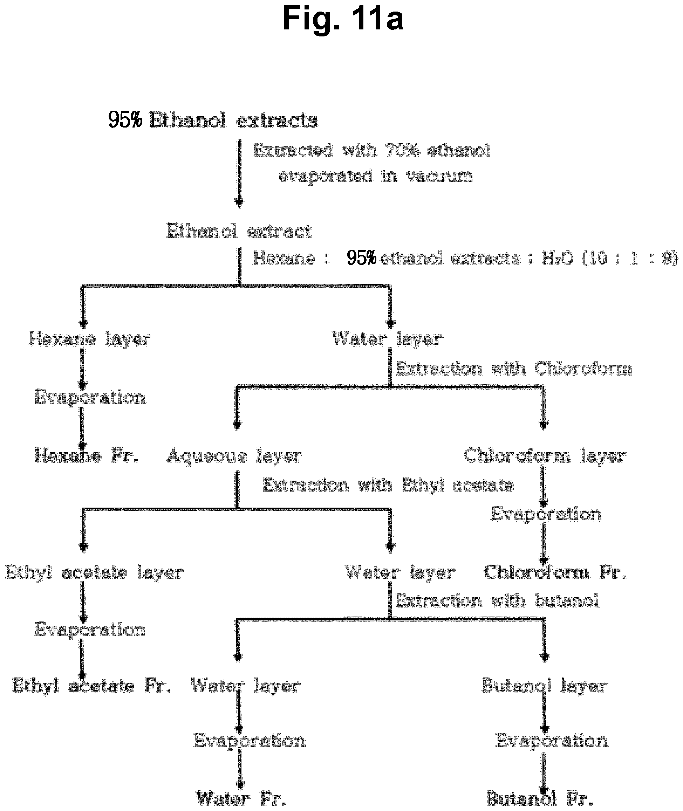

Fig. 11a shows the separation process of 95% ethanol extract of *Poria cocos* Wolf bark into hexane fraction, chloroform fraction, ethyl acetate fraction, butanol fraction and water fraction. Fig, 11b shows that the hexane fraction of *Poria cocos* Wolf bark extract has foxp3 induction activity.

## DETAILED DESCRIPTION OF THIS INVETNION

In one aspect of this invention, there is provided a composition for use in preventing, improving, or treating Th2-mediated immune diseases comprising *Poria cocos* Wolf bark extract as active ingredient.

The present inventors have endeavored to develop natural products that may prevent or treat diseases and symptoms caused by Th1 or Th2 immune responses such as inflammatory diseases and allergies. As a result, the inventors have successfully completed this research by finding out that *Poria cocos* Wolf bark extract inhibits production of Th1-related cytokines and Th2-related cytokines, penetration of allergens through intestinal epithelial cells, degranulation of mast cells; induces differentiation into Treg cells; and stabilizes Treg cells.

The *Poria cocos* Wolf bark extract can be obtained by using various extraction solvents. The extract solvents can be polar solvents or non- polar solvents. The polar solvents include (i) water, (ii) alcohols (preferably, methanol, ethanol, propanol, butanol, normal propanol, iso- propanol, normal butanol, 1-pentanol, 2-butoxy ethanol or ethylene glycol), (iii) acetic acid, (iv) DMFO (dimethyl-formamide) and (v) DMSO(dimethyl sulfoxide). The non-polar solvents include acetone, acetonitrille, ethyl acetate, methyl acetate, fluoroalkanes, pentan, hexan, 2,2,4-trimethylpentan, trimethylpentan, decans, cyclohexan, cyclopentan, diisobutylene, 1-pentene, 1-chlorobutan, 1-chloropentan, o-xylene, diiospropyl ether, 2-chlorobutan, toluene, 1-chloropropan, chlorobenzene, benzene, diethyl ether, diethyl sulfide, chloroform, dichloromethane, 1,2-dichloroethane, aniline, diethylamine, ether, CC14 and THF (tetrahydrofuran).

The extraction solvents used are (a) water, (b) anhydrides of carbon number 1 through 4 or enhydrous alcohol with a low number of molecules (methanol, ethanol, propanol, butanol, etc.), (c) mixed solvents of the aforementioned alcohol and water, (d) acetone, (e) ethyl acetate, (f) chloroform, (g) butyl acetate, (h) 1,3-butylene glycol, (i) hexane and (j) diethyl ether. Also, the extracts are obtained by applying water, ethanol or the combination to *Poria cocos* Wolf bark extract, *Cynanchym atratum* Bunge extract or *Arisaema amurense* extract.

The term used herein "extract" is widely used to mean crude extracts, while a wider meaning also refers to additional materials fractionated from these extracts. That is, *Poria cocos* Wolf bark extract includes, in definition, not only the extraction solvents previously explained but also any extracts acquired by applying an additional refinement process. For instance, there is fractionation obtained by penetrating the above extracts through an ultrafilter that retains constant cut-off values for the number of molecules; another example is the differentiation based on various chromatographies (those manufactured for differentiation by size, electric charge, hydrophobicity or hydrophilicity). Also, these fractionation, which has been acquired by many different refinement procedures carried out additionally, are included in *Poria cocos* Wolf bark extract.

These extracts can be manufactured in a powdery state through an additional process consisted of vacuum distillation, freeze-drying or spray drying.

The term used herein "comprising as active ingredients" refers to the case in which a sufficient amount of extracts are contained in order to maximize their effects or achieve activation. This invention is a composition of the extracts from natural plant materials including *Poria cocos* Wolf bark extract, which leaves no side effects in a human body even when too much of it is administered. Therefore, the person in the art may determine the maximum quantity of these extracts within a proper range.

The compositions of the present invention induce the differentiation into Treg cells and are effective in preventing, improving or curing Th1- and/or Th2-mediated immune diseases. The transformation from T cell to Treg cell can be seen from the increasing expression of Foxp3, and this induction of Treg cells brings about the balance between Th1 and Th2 and is effective in preventing, improving or curing Th1- and/or Th2-mediated immune diseases.

According to an embodiment of the present invention, *Poria cocos* Wolf bark extract induces the differentiation into Treg cells.

According to the present disclosure, *Poria cocos* Wolf bark extract of the present invention increases the expression of Fox3p by 1.5-5.0, 1.5-3.0, 2.0-3.0 or 2.5-3.0 times, compared to a control group.

The increasing expression of Foxp3 means that *Poria cocos* Wolf bark extract of the present invention induces differentiation into Treg cell.

According to an embodiment of the present invention, *Poria cocos* Wolf bark extract of the present invention increases the production of TGF-β. According to the present disclosure, the *Poria cocos* Wolf bark extract increases the production of TGF-β by 30-90%, 50-90%, 60-90%, 60-80% or 65-75%, compared to a control group.

The term used herein "stabilizing activity of Treg cells" represents the activities of Treg cells are maintained.

According to an embodiment of the present invention, *Poria cocos* Wolf bark extract has Treg cells stabilizing activity.

According to the present disclosure, a ratio of foxp3 expressing cells is 65-90% at three days after Treg cells cultivation in case of *Poria cocos* Wolf bark extract treatment. According to the present disclosure, the ratio is 67-85%, 67-80%, 67-77%, 70-77% or 72-77%.

The ratio of foxp3 expressing cells represents stabilizing activity of Treg cells of *Poria cocos* Wolf bark extract by maintenance of Treg cell activity.

According to other embodiment of the present invention, a ratio of foxp3 expressing cells increases 3-25% at three days after Treg cells cultivation in case of *Poria cocos* Wolf bark extract treatment, compared to a control group. According to the present disclosure, the ratio is 3-20%, 3-17%, 5-17%, 5-13%, 7-13% or 9-13%.

The increase of foxp3 expressing cell ratio represents that *Poria cocos* Wolf bark extract has activity of Treg cells.

According to the present disclosure, *Poria cocos* Wolf bark extract used in Treg cell induction or stabilization of the present invention has concentration of 2-30, 5-30, 7-30, 9-30, 9-20, 9-15 or 9-12 mg/ml. According to the present disclosure, *Poria cocos* Wolf bark extract used in Treg cell induction or stabilization of the present invention has concentration of 2-30, 2-20, 5-20, 7-20, or 7-15 mg/ml.

The term used herein "Th1-mediated immunity diseases" refers to the diseases involved with Cytokines, such as IL-1β, IL-2, IL-12, IL-17, IFN-γ or TNF-α, which are created by the formation and/or activation of Th1 cells. IL-17 is produced by Th17 cells (CD4$^+$T cells) and TNF-α, IL-1β, IL-6 and IFN-γ etc. are involved in IL-17 production. Because IL-17 production is induced by the cytokines, IL-17 is involved in Th1-mediated immunity diseases.

The term used herein "Th1 cells" represents the subset of helper T cell lymphocytes specified for the purposes of gene expression, protein secretion and functional activation. For example, Th1 cells manifest a cytokine expression pattern, which synthesizes IL-2 and IFN-γ but does not synthesize IL-4, IL-5, IL-10 and IL-13. Also, the Th1 cells are involved in cell-mediated immune responses on various pathogens within cells, organ-specific autoimmune diseases as well as delayed hypersensitive reactions.

There are no specific restrictions on the Th1-mediated immunity diseases. According to the present disclosure, the Th1-mediated immunity disease, to which the compositions of the invention are applied, is transplant rejection, autoimmune diseases or inflammatory diseases. According to the present disclosure, Th1-mediated immunity disease, is colitis, inflammatory bowel disease, Type I diabetes, Type II diabetes or rheumatoid arthritis.

Th1-mediated immunity diseases include colitis, inflammatory bowel disease, Type I diabetes, Type II diabetes, rheumatoid arthritis, reactive arthritis, osteoarthritis, psoriasis, scleroderma, osteoporosis, atherosclerosis, myocarditis, endocarditis, pericarditis, cystic fibrosis, Hashimoto's thyroiditis, Graves' disease, leprosy, syphilis, Lyme disease, borreliosis, neurogenic borreliosis, tuberculosis, sarcoidosis, lupus, discoid lupus, chilblain lupus, lupus nephritis, systemic lupus erythematosus, asthma, macular degeneration, uveitis, irritable bowel syndrome, Crohn's disease, Sjogren's syndrome, fibromyalgia, chronic fatigue syndrome, chronic fatigue & immunological incompetence syndrome, myalgic encephalomyelitis, amyotrophic lateral sclerosis, Parkinson's disease, multiple sclerosis, autism spectrum disorder, attention deficit disorder, attention deficit and hyperactivity disorder, but are not limited thereto. According to the present disclosure, the Th1-mediated immunity disease is colitis, inflammatory bowel disease or rheumatoid arthritis.

The composition of this invention may be used to prevent or treat various Th2-mediated immunity diseases, disorders or symptoms, wherein the TH2-mediated diseases are allergic diseases.

The term used herein "Th2-mediated immunity diseases" refer to the diseases involved with IgE and mast cells based on production and activity of allergen-specific Th2 cells.

The term used herein "Th2 cells" represents the subset of helper T cell lymphocytes specified for the purposes of gene expression, protein secretion and functional activation. Th2 cells manifest a pattern of IL-4, IL-5, IL-10 and IL-13 Cytokines, and are involved in humoral immunity reactions.

The type of Th2-induced immunity diseases is allergic diseases.

The term used herein "allergy" means various diseases and symptoms induced by a human immune system's hypersensitive reactions against a certain matter coming from outside. Allergic diseases that apply to the compositions of this invention are, preferably, Type I immediate hypersensitive reactions and Type IV delayed hypersensitive reactions. Type I immediate hypersensitive reactions include bronchial asthma, allergic rhinitis, atopic dermatitis, allergic conjunctivitis, allergic otitis media, hives and anaphylactic shocks, whereas some of the Type IV delayed hypersensitive reactions are contact hypersensitivity, allergic contact dermatitis, bacteria allergies, fungus allergies, virus allergies, and drug allergies, thyroiditis and allergic encephalitis. Type I immediate hypersensitive reactions are divided into 2 steps. In the first step, allergen penetrates into a body and inhibits secretion of IgE and IgG1. Once the balance between Th1 cellular response, which produces IL-12 and IFN-$\gamma$ that increase secretion of IgG2a, and Th2 cellular response, which produces IL-4, IL-5 and IL-13, tips in the direction of Th2, IL-4, IL-13 and others are discharged due to an excessive hypersensitive reaction of Th2. Because of this effect, IgE specific antibodies produced by B cells are attached to mast cells or basophils, and, subsequently, an allergic crisis is ready, all of which is called the sensitization of allergens. The second step of allergic crisis is divided into early and late responses. In the early response, an allergen re-penetrates the body, stimulates mast cells and induces degranulative reactions, releasing histamines, lipid metabolites and cytokine at this time to cause blood vessels to expand. In the late response, neutrophils, eosinophils, macrophage, Th2 cells and basophils infiltrate into a particular tissue and are activated, which leads to inflammation and symptoms of atopic dermatitis, rhinitis and asthma. Among these secreted matters of degranulation, histamine is the most well-known and used as an essential indicator for allergic symptoms due to its relations to immediate hypersensitive reactions.

The allergic diseases to which the invented compositions are applied include atopic dermatitis, other dermal diseases related to atopic syndrome, allergic rhinitis (acute or chronic), hay fever, asthma and food allergies, but not limited thereto. According to the present disclosure, the allergic diseases to which this invention is applied include asthma, allergic rhinitis, allergic dermatitis, allergic atopic dermatitis or food allergies. According to the present disclosure, the allergic diseases are food allergies.

According to an embodiment of the present invention, the compositions of the invention inhibit degranulation of mast cells.

The term used herein "degranulation" refers to a process in which antibacterial cytotoxic molecules are separated from granules and secretory vesicles existing inside a specific cell, which includes granulocytes (neutrophils, eosinophils and basophils) and mast cells inside the immune system as well as specific lymph cells such as natural killer cells and cytotoxic T cells.

According to the present disclosure, *Poria cocos* Wolf bark extract of the present invention inhibits the degranulation of mast cells by 5-95%, 5-80% or 7-80%, compared to a control group. According to the present disclosure, *Poria cocos* Wolf bark extract of the present invention inhibits the degranulation of mast cells by 10-90% or 10-80%, compared to a control group. According to the present disclosure, *Poria cocos* Wolf bark extract of the present invention inhibits the degranulation of mast cells by 50-95%, 50-90%, 60-90% or 60-80%. According to the present disclosure, *Poria cocos* Wolf bark extract used for inhibition of the mast cell degranulation of the present invention has concentration of 0.005-10, 0.007-10, 0.3-10, 0.5-10, 0.8-10, 0.8-5, 0.8-3, 0.8-2, 0.8-1.5 or 0.8-1.2 $\mu$l/ml. According to the present disclosure, *Poria cocos* Wolf bark extract used for inhibition of the mast cell degranulation of the present invention has concentration of 0.3-5, 0.5-5, 0.5-3, 0.5-2 or 0.5-1.5 $\mu$l/ml.

According to an embodiment of the present invention, *Poria cocos* Wolf bark extract of the present invention inhibits the production of one or more Th2-related cytokines selected from the group consisting IL-4, IL-5, IL-10 and IL-13.

According to the present disclosure, *Poria cocos* Wolf bark extract of the present invention inhibits the production of IL-4 by 10-99%, 20-99%, 30-99%, 40-99%, 40-90%, 40-80%, 40-70%, 40-60% or 40-50%, compared to a control group. According to the present disclosure, *Poria cocos* Wolf bark extract of the present invention inhibits the production of IL-4 by 50-99%, 50-90%, 50-80%, 60-80% or 60-70%, compared to a control group. According to the present disclosure, *Poria cocos* Wolf bark extract of the present invention inhibits the production of IL-4 by 50-99%, 60-99%, 70-99%, 80-99% or 80-90%, compared to a control group. According to the present disclosure, *Poria cocos* Wolf bark extract used for inhibition of the IL-4 production of the present invention has concentration of around 2-50, 5-50, 7-50, 7-40 or 7-35 mg/ml. According to the present disclosure, *Poria cocos* Wolf bark extract used for inhibition of the IL-4 production of the present invention has concentration of around 2-30, 2-20, 5-20, 7-20, 7-15 or 9-12 mg/ml. According to the present disclosure, *Poria cocos* Wolf bark extract used for inhibition of the IL-4 production of the present invention has concentration of around 9-50, 15-50, 20-50, 20-40, 25-40, 25-35 or 28-32 mg/ml. According to the present disclosure, *Poria cocos* Wolf bark extract used for inhibition of the IL-4 production of the present invention has concentration of around 5-50, 10-50, 10-40, 20-40, 20-35, 20-30 or 23-27 mg/kg (body weight).

According to the present disclosure, *Poria cocos* Wolf bark extract of the present invention inhibits the production of IL-5 by 20-90%, 30-90%, 30-80%, 30-70%, 30-60%, 30-50% or 40-50%, compared to a control group. According to the present disclosure, *Poria cocos* Wolf bark extract of the present invention inhibits the production of IL-10 by 20-99%, 30-99%, 30-99%, 40-99%, 40-90%, 40-80%, 40-70%, 40-60% or 50-60%, compared to a control group. According to the present disclosure, *Poria cocos* Wolf bark extract of the present invention inhibits the production of IL-10 by 50-99%, 60-99%, 70-99%, 80-99% or 90-99%, compared to a control group. According to the present disclosure, *Poria cocos* Wolf bark extract of the present invention inhibits the production of IL-13 by 5-50%, 5-40%, 5-30%, 10-30%, 10-25% or 15-20%, compared to a control group.

According to an embodiment of the present invention, *Poria cocos* extract of the present invention inhibits the production of one or more Th1-related cytokines selected from the group consisting of IL-17 and IFN-γ. According to the present disclosure, *Poria cocos* extract of the present invention inhibits the production of IL-17 by 20-90%, 30-90%, 30-80%, 40-80%, 40-70%, 40-60% or 45-55%, compared to a control group. According to the present disclosure, *Poria cocos* extract of the present invention inhibits the production of IFN-γ by 20-99%, 30-99%, 40-99%, 50-99%, 60-99%, 70-99%, 80-99% or 90-99%, compared to a control group. According to the present disclosure, *Poria cocos* Wolf bark extract used for inhibition of IL-17 production of the present invention has concentration of around 2-30, 2-20, 5-20, 7-20, 7-15 or 9-12 mg/ml.

According to an embodiment of the present invention, *Poria cocos* Wolf bark extract of the present invention inhibits the production of one or more antibodies selected from the group consisting of IgE, IgG2a and IgG1 in serum. According to the present disclosure, *Poria cocos* Wolf bark extract of the present invention inhibits the production of IgE by 10-70%, 10-60%, 20-60%, 20-50%, 30-50%, 30-45%, 35-45% or 35-40%, compared to a control group. According to the present disclosure, *Poria cocos* Wolf bark extract of the present invention inhibits the production of IgG2a by 10-70%, 10-60%, 20-60%, 20-50%, 30-50%, 30-45%, 35-45% or 35-40%, compared to a control group. According to the present disclosure, *Poria cocos* Wolf bark extract of the present invention inhibits the production of IgG2a by 10-80%, 20-80%, 30-80%, 30-70%, 40-70%, 40-60% or 45-55%, compared to a control group.

According to the present disclosure, *Poria cocos* Wolf bark extract used for inhibition of IgE, IgG2a, IgG1, IL-5, IL-10, IL-13, INF-γ or TGF-β production has concentration of around 5-50, 10-50, 10-40, 20-40, 20-35, 20-30 or 23-27 mg/kg (body weight).

Compositions for use in preventing, improving, or treating Th2-mediated immune diseases comprising as active ingredient *Poria cocos* Wolf bark extract are pharmaceutical, food, functional food, or fodder compositions.

Compositions of this invention can be manufactured as pharmaceutical.

According to the present disclosure, the compositions of the present invention are pharmaceutical compositions comprising (a) a pharmaceutically effective amount of *Poria cocos* Wolf bark extract and (b) a pharmaceutically accepted carrier. In this specification, the term "pharmaceutically effective amount" refers to a sufficient amount needed to achieve effectiveness or activation of *Poria cocos* Wolf bark extract.

If the compositions of this invention are manufactured as pharmaceutical compositions, they can include pharmaceutically accepted carriers. These carriers, which are normally used in manufactured medicine, include, but are not limited to, lactose, dextrose, sucrose, sorbitol, mannitol, starch, acacia rubber, calcium phosphate, alginate, gelatin, calcium silicate, microcrystalline cellulose, polyvinyl pyrrolidone, cellulose, water, syrup, methyl cellulose, methyl hydroxybenzoate, profile hydroxybenzoate, talc, stearic acid magnesium and mineral oil. Also included in pharmaceutical compositions are lubricants, humectants, sweetening agents, cordials, emulsifiers, suspensions and preservatives. Proper carriers and medication pharmaceutically accepted are written in detail in Remington's Pharmaceutical Sciences (19th ed., 1995).

The pharmaceutical compositions of this disclosure may be administered by either orally or non-orally; in this application, an oral administration is performed.

The appropriate dosage of the pharmaceutical composition of the present disclosure is varied depending on factors, such as the method of formulation, the manner of administration, the age, body weight, sex, morbidity, and diet of the patient, the time of administration, the route of administration, the rate of excretion, and response sensitivity. According to the present invention, the appropriate dosage per day is 0.001-100 mg/kg (body weight).

The pharmaceutical composition of the present disclosure may be formulated into a unit dosage form or may be formulated by being contained in a multi-dose container, using a pharmaceutically acceptable carrier and/or excipient, according to the method easily performed by person having ordinary skills in the art to which the present invention pertains. Here, the dosage form may be a solution in an oily or aqueous medium, a suspension, a syrup, or an emulsion, or an extract, a powder, a granule, a tablet, or a capsule, and may further include a dispersant or a stabilizer.

Compositions of this invention can be manufactured as a food composition. If the compositions of this invention are manufactured as compositions for preventing, improving, or treating Th2-mediated immune diseases, they can include additional ingredients which are used in food manufacturing, such as proteins, carbohydrates, lipids, nutrients, seasonings and flavours. Carbohydrates include monosaccharides such as glucose, fructose etc.; disaccharides such as maltose, sucrose, oligosaccharides, etc.; and polysaccharides such as dextrin, cyclodextrin as a normal saccharides and xylitol, sorbitol, erythritol as a sugar alcohol. When these compositions are manufactured as food compositions, ingredients normally added during food manufacturing are included while both natural (thaumatin and stevia extracts (for example, rebaudioside A, glycyrrhizin, etc.)) and synthetic (saccharin, aspartame, etc.) cordials may be used, on top of the aforementioned natural products. Moreover, if the food compositions of this invention are manufactured as tonics, the following can be added on top of the aforementioned natural products: citric acid, high fructose corn syrup, sugar, glucose, acetic acid, malic acid, fruit juice, eucommia bark extracts, jujube extracts and licorice extracts.

Compositions of this invention can be manufactured as a functional food composition. If the compositions of this invention are manufactured as functional food compositions, ingredients normally added during food manufacturing such as protein, carbohydrate, fat, nutrients and flavoring agents are included. For example, the natural carbohydrates include monosaccharides (e.g. glucose, fructose); disaccharides (e.g. maltose, sucrose); oligosaccharides; poly saccharides (e.g. dextrin, cyclodextrin); and saccharide alcohols (e.g. xylitol, sorbitol, erythritol. Flavoring agents include natural flavoring agents (e.g. taumarin, stevia extracts) and synthetic flavoring agents (e.g. sacarine, aspartam).

The solution or emulsion formulations may include solvents or emulsifying agents as carriers such as water, ethanol, isopropanol, ethyl carbonate, ethyl acetate, benzyl alcohol, benzyl benzoate, propylene glycol, 1,3-butylglycol oil, glycerol aliphatic ester, polyethylene glycol or fatty acid ester of sorbitan.

If the type of this invention is a suspension, the following may be used as a carrier ingredient: diluting agents such as water, ethanol and propylene glycol, suspensions such as ethoxylated isostearyl alcohol, polyoxyethylene sorbitol ester and polyoxyethylene sorbitan ester, microcrystalline cellulose, aluminum metal hydroxides, bentonite, agar and tragacanth.

If the type of this invention is a surfactant-containing cleansing, it can include aliphatic alcohol sulfate, aliphatic alcohol ether sulfate, sulfosuccinate monoester, isethionate, imidazolium derivates, methyl taurate, sarcosinate, fatty acid amide ether sulfate, alkylamidobetaine, aliphatic alcohols, fatty acid glyceride, fatty acid diethanolamide, plant oils, lanolin derivates or ethoxyfying glycerol fatty acid ester as carriers.

Compositions of this invention can be manufactured as a fodder composition. The fodder composition can additionally include *Cynanchym atratum* Bunge extract or *Arisaema amurense* extract as raw materials, or additionally include additives such as vitamins, amino acids, minerals, antioxidants, antibiotics, antimicrobial agents or other additives. The additives can be powders, granules, pellets or suspensions.

If the compositions of this invention are manufactured as fodder compositions, they can be supplied either independently or as a mixture with fodder for both land and sea animals. In this case, the types of fodder include, but are not limited to, powder food, solid food, moist pellet food, dry pellet food, EP (Extruder Pellet) food and raw food.

The features and advantages of this invention will be summarized as follows:

(a) The present invention provides compositions for use in preventing, improving, or treating Th2-mediated immune diseases, comprising *Poria cocos* Wolf bark extract.

(b) The compositions of this invention have excellent inhibitory activities of Th1-related cytokines and Th2-related cytokines production, degranulation of mast cells, penetration of allergens through an intestinal epithelial cell layer, differenciation inducing activity into Treg cells and stabilizing activity of Treg cells.

(c) This disclosure provides detailed mechanism of action for anti-allergy materials in natural products and foods.

(d) This invention uses natural products for its materials in order to provide stability, reduction in manufacturing costs and convenience of usage.

## Examples

### Example 1: Preparing Samples and Solvent Extracts

*Poria cocos* Wolf bark extract (invention), *Cynanchym atratum* Bunge extract (for comparison only) and *Arisaema amurense* extract (for comparison only) were purchased from Korea Plant Extract Bank and used as samples, each of which was extracted with 95% ethanol or water.

That is, a 200 g sample powder was added to 1.8 L of 95% EtOH, and the first extraction was performed via reflux extraction for 3 hours. Then extracts were performed vacuum filtration, and samples that had remained after filtration were added to 2 L of 95% ethanol followed by the second extraction, obtaining 95% ethanol extracts of the samples. The 95% ethanol extraction temperature of the samples is 84-86°C. The results of the first extract and the second extract was combined, and enriched followed by freeze drying.

**Example 2: Test for inhibitory activity for penetration of OVA using Caco-2 single cell layer**

*Caco-2 cell culture and test for inhibitory activity for penetration of OVA*

Caco-2 cell strains (HTB-37) originated from ATCC underwent subculture in a DMEM (Dulbeco's Modified Eagle's Medium) medium, which contains 10% FBS, 1% 100 U/ml penicillin, 100 μg/ml streptomycin and 0.1 mM non-essential amino acid under the conditions of 37°C and 5% $CO_2$. In order to conduct a passing test of food allergens using a Caco-2 single cell layer, 0.5 ml of Caco-2 cells were deposited on the transmembrane, which forms the apical side of 12 transwell plates, at the concentration of $2 \times 10^5$ cells/ml by using the above culture medium. On the basolateral side of a well, 1.5 ml of the culture medium was added and cultivated in a thermo-hygrostat under the conditions of 37°C and 5% CO2. The medium was changed once every 2-3 days. In 2-3 weeks after the deposition, Caco-2 cells came to form a single cell layer. Here, the Transepithelial Electrical Resistance (TEER, $\Omega \times cm^2$) between basolateral and apical sides of the Caco-2 cells were measured using the Millicell-ERS (Electrical Resistance System, Millipore, U.S.A.), and cells whose values are greater than 300 $\Omega \times cm^2$ were used in experiments. The Caco-2 single cell layer was then washed with HBSS (Hank's Balanced Salt Solution) for 3 times, and *Cynanchym atratum* Bunge extract or *Arisaema amurense* extract (final concentration: 400 μg/ml) was added to the apical side, along with bile salts (final concentration: 100-150 μg/ml). The mixture was then processed for 1 hour in a thermo-hygrostat under the conditions of 37 °C and 5% $CO_2$. Next, OVA (Ovalbumin) was added to the apical side (final concentration: 400 μg/ml), and the mixture was placed in the identical thermo hygrostat with same conditions to react for 3 hours, after which the TEER value was measured one more time.

The experimental group which was treated with both of *Arisaema amurense* extract and OVA showed that TEER levels were higher than the positive control which was treated with only OVA. This result represents that the *Arisaema amurense* extract inhibits the penetration of allergens through the intestinal epithelial cell layer by increasing TEER levels of Caco-2 single cell layer (fig. 1c).

Here, succus on the basolateral side was gathered and the concentration of passed OVA was measured using sELISA (sandwich Enzyme-Linked Immunosorbent Assay). The amount of passed OVA contained within the basolateral side can be calculated as follows.

$$\texttt{Flux = C ng/ H hr/ S cm}^2$$

In the above equation, C represents the amount of passed OVA calculated by using ELISA, H the reaction time after adding OVA in the apical side of the Caco-2 single cell layer (3h), and S the apical side's surface area on a transwell plate (1.12 cm2).

On investigation of the OVA penetration amount, the experimental group which was treated with both of *Arisaema amurense* extract and OVA showed that the OVA penetration amounts were reduced, compared to the positive control which is treated with only OVA. On converting the result into a percentage, the effects of the OVA penetration inhibition were 40-60% by the Cynanchym atratum Bunge extract and 10-20% by the Arisaema amurense extract (fig. 1b and 1c).

*OVA analysis by ELISA*

Anti-rabbit-OVA antibodies diluted to the concentration of 2 μg/ml was combined with a coating buffer solution ((tris hydroxymethyl) aminomethane 0.05 M, pH 9.0), and 100 μl of the mixture was deposited onto 96 well plates and kept overnight at 4°C. The coated 96 well plates were washed 3 times with a washing buffer solution (PBS (Phosphate Buffered Saline) containing Tween 20; PBST, 0.138 M NaCl, 0.0027 M KCl and 0.01 M phosphate buffer solution containing 0.05% Tween 20). Then, 100 μl of standard OVA, which had been diluted by a specific multiple, and the recovered solution were added to HBSS for each well, and were left to react at room temperature for 1 hour. The well plates were washed again with the washing buffer solution, and biotin, which had been bound with anti-OVA antibodies, was diluted to a certain concentration by PBST. Then, 100 μl for each well were processed and left to react at room temperature for 1 hour. Repeating the above process with a different solution this time, the plates were washed with the washing buffer solution, avidin-HRP was diluted to a certain concentration by PBST and 100 μl of the mixture were processed for each well and left to react at room temperature for 1 hour. Then, another washing operation took place by the washing buffer solution. Next, for a substrate solution, phenyl propionic acid/phosphate buffer (36 mM, pH 7.0) added with 0.002% $H_2O\square$ was used while, for a response stop solution, 100 μl of 1 M NaOH-glycine (1 M glycine/1 M NaOH) was used for each well. A fluorescent microplate reader was used to measure the fluorescence at 320/405 nm (Ex./Em. wavelength).

**Example** 3: **Testing of Th1/Th2 Cytokine Balance Control Activated by Extracts (ex *vivo*)**

*Mouse spleen cell culture immunized with OVA*

Allergens were administered to 5-week-old female BALB/c mice twice in the span of a week through intraperitoneal immunization in order to induce allergies. The allergens, a solution of OVA (20 $\mu$g) and alum (2 mg) mixed for 30 minutes, were intraperitoneally injected for 100 $\mu$l on each mouse (n=5). After a week of immunization, the cervical vertebrae of the mice were dislocated, their spleens were extracted under sterile conditions, transferred to a small petri dish containing the basal medium of 1 ml (RPMI1640 containing 2-mercaptoethanol and antibiotics, WelGene, Daegu, Korea), and subsequently kept on top of an ice box. The spleens contained in a petri dish, were monocellularized by using a mesh and washed twice with 5 ml of basal medium before performing a centrifugation at 1500 rpm for 5 minutes. After removing the supernatant, 1 ml of RBC (Red Blood Cell) buffer solution (Sigma R7757, U.S.A.) was added, shaken sufficiently by hand and suspended. It was then left on ice for 3 minutes and another 10 ml of basal medium was added and suspended before performing 2 times of centrifugation at 1500 rpm for 5 minutes. Antigens (OVA, 100 $\mu$g/ml) were processed on a 96-well plate and specific concentrations for each of the aforementioned natural products were added. Then, the spleen cells were separately deposited on the well at $5 \times 10^6$ cells. The supernatant was collected after 72 hours of incubation in a thermal hygrostat with 37°C, $CO_2$.

*Analyzing cytokine of spleen cell culture supernatant*

The spleen cell culture supernatants which were treated with the antigen (OVA, 100 $\mu$g/ml) and extract were collected in 72 hours and used in cytokine analysis. The analysis was performed by using an BD OptEIATM MOUSE ELISA (Enzyme-linked Immunosorbent Assay) according to the protocol of the manufacturer. Briefly, captured antibodies were added onto 96 well plates, which were placed overnight at 4°C. The 96 well plates coated with the captured antibodies were then washed with a washing buffer solution (PBST), and 200 $\mu$l of diluent for analysis (PBS containing 10% FBS) was added to each well before undergoing blocking for 1 hour at room temperature. Next, 100 $\mu$l of each standard specimen and sample was deposited onto each well and left to react for 2 hours at room temperature, and 100 $\mu$l of each detection antibody and streptavidin-HRP was deposited onto each well and left to react for 1 hour at room temperature. After reaction, each well was driven to develop a certain color, with the help of 100 $\mu$l of substrate solution (citric acid-phosphate buffer containing 0.01% TMB, pH 5.0, 0.001% $H_2O_2$), for 30 minutes at room temperature. Then, 50 $\mu$l of 2M $H_2SO_4$ was added to each well in order to stop the color reaction. The level of color formation was gauged with a microplate reader (THERMOmax, Molecular Devices, U.S.A.) by measuring the absorbance at 450 nm.

An investigation of the effects that *Poria cocos* Wolf bark extract or *Cynanchym atratum* Bunge extract has on cytokine production of spleen cells showed that the *Poria cocos* Wolf bark extract reduced the IL-4 production in spleen cells by 65.1% at the concentration of 10 $\mu$g/ml and 86.5% at the concentration of 30 $\mu$g/ml, compared to the control group, the IL-10 production by 95% (fig. 2a), the IFN-$\gamma$ production by 95.8% and the IL-17 production by 50.5% and (fig. 2b). Also, the *Cynanchym atratum* Bunge extract reduced the IL-4 production in spleen cells by 47.2% at the concentration of 10 $\mu$l/ml, compared to a control group (Fig. 2c).

The reducing effects of IL-4 and/or IL-10 production of Poria cocos Wolf bark extract or Cynanchym atratum Bunge extract shows that the *Poria cocos* Wolf bark extract or the *Cynanchym atratum* Bunge extract reduces Th2 immune response.

Also, the reducing effects of IL-17 and/or IFN-$\gamma$ production of *Poria cocos* Wolf bark extract shows that the *Poria cocos* Wolf bark extract reduces Th1 immune response, Th17 immune response or Th1/Th17 immune response.

**Example 4: Test for inhibitory activity of degranulation**

*Collecting mast cells in abdominal cavity*

After anesthetizing a mouse with ether and killing it by heavily striking the back of its head, about 10 ml of medium (pH 7.4) was injected into its abdominal cavity and the abdominal wall was gently massaged for 90 seconds before being cut at the center line. An abdominal washing solution was collected via pipettes and, after allowing it to undergo centrifugation at $100 \times$g for 10 minutes, the supernatant was removed. The abdominal suspension was made and utilized in experiments in such a way that the number of mast cells becomes $1 \times 10^6$ cells/ml in the identical medium.

*Observing morphologic alteration of mast cells*

25 $\mu$l of saline or 25 $\mu$l of the *Poria cocos* Wolf bark extract, *Cynanchym atratum* Bunge extract or *Arisaema amurense* extract (each of 1 $\mu$g/ml, 0.1 $\mu$g/ml and 0.01 $\mu$g/ml) was added to 200 $\mu$l of mast cell suspension which had been re-suspended, and was left to react for 10 minutes inside a 37°C-incubator. After the reaction was complete, a 25-μl solution of Compound 48/80 (Sigma Chemical Co., St. Louis, MO, U.S.A.) was added and left to react for 20 minutes.

To observe the shape of mast cells through an optical microscope, 200 μl of the mast cell suspension in which the reaction had been completed was dropped on top of a slide glass (slide glass, 22×60 mm) located on the stage of an inverted microscope, allowing the mast cells to be precipitated and placed at room temperature for 10 minutes. By using a hemacytometer with four hundred-fold magnification, the mast cells were observed through an inverted microscope (Olympus, Japan). Being mostly round-shaped or egg-shaped, mast cells have a defined cell outline and are filled with a lot of granules inside their cytoplasm. The diameter of a mast cell is about 10-20 μm, which is twice as large as those of other cells (lymphocytes or neutrophil leukocytes) in an abdominal suspension. Thus, cells that possess these characteristics are categorized as normal mast cells. In contrast, cells that have a vague outline and/or granules within cytoplasm either sticking out of the surface or dispersed around them are regarded as degranulated type. The rate of degranulation was calculated based on the following formula.

```
Rate of degranulation of mast cells (%) = (the
number of degranulated mast cells / the total number of
mast cells) x 100
```

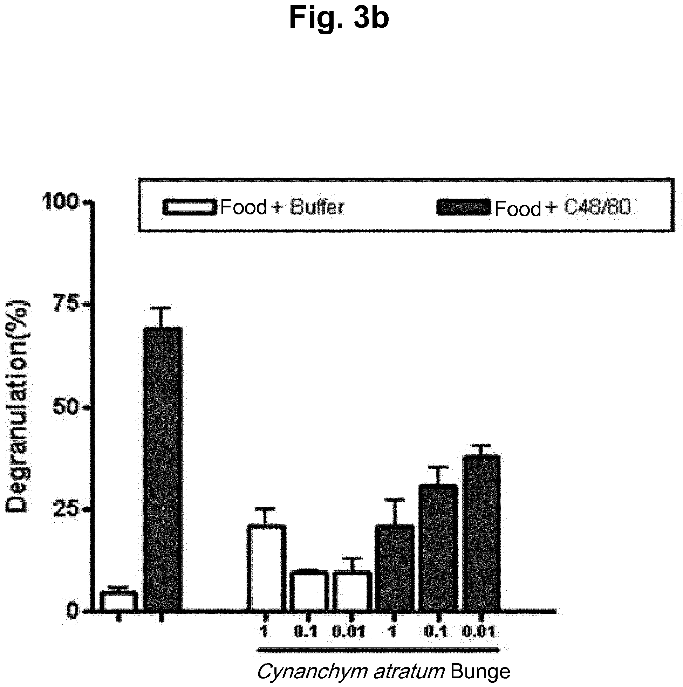

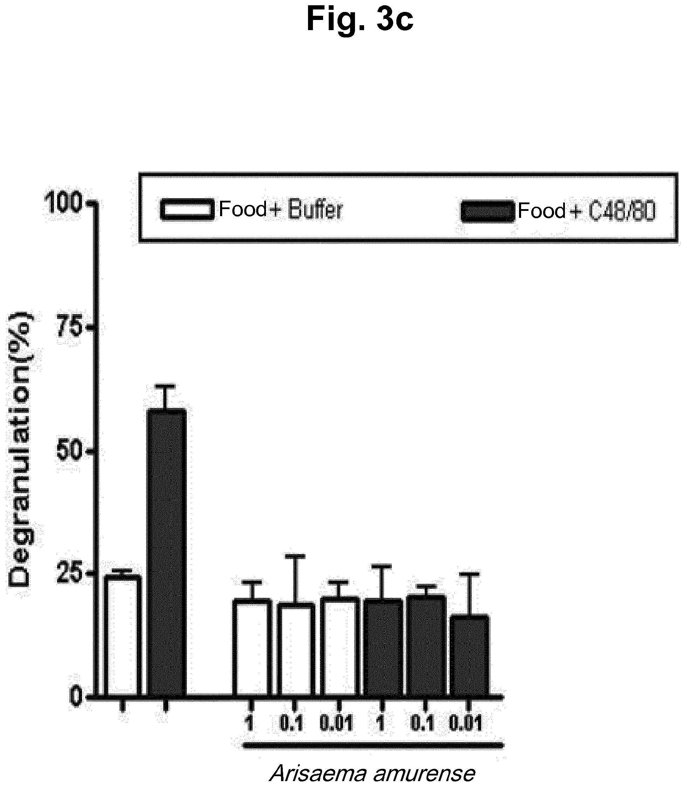

The effects that *Poria cocos* Wolf bark extract, *Cynanchym atratum* Bunge extract or *Arisaema amurense* extract have on the degranulation rate of mast cells due to Compound 48/80 were examined. Compared to the control group that only processed Compound 48/80, adding 1.0 μg/ml of *Poria cocos* Wolf bark extract to a test group that had been processed with Compound 48/80 turned out to show effects of degranulation inhibition by 65-85% (fig. 3a). And if 0.01-1.00 μg/ml of *Cynanchym atratum* Bunge extract is processed, the effect of degranulation inhibition was 40-80% (fig. 3b). Also, if 0.01-1.00 μg/ml of *Arisaema amurense* extract is processed, the effect of degranulation inhibition was 50-70% (fig. 3c).

**Example 5: Induction activity into Treg cells**

*Experimental animals*

6- to 8-week-old female BALB/c mice were purchased from Charles River Laboratories. They were raised in an animal testing facility located at the College of Pharmacy, Seoul National University, and all tests were conducted according to the regulations and guidelines established by the Institutional Animal Care and Use Committees at Seoul National University.

*Production of Poria cocos Wolf bark extract, Cynanchym atratum Bunge extract or Arisaema amurense extract*

Each of *Poria cocos* Wolf bark extract, *Cynanchym atratum* Bunge extract or *Arisaema amurense* extract was provided by Korea Food Research Institute as dissolved in DMSO (Dimethyl Sulfoxide) at the concentration of 25-100 mg/ml. In order to create a concentration suitable for the experiment, the above extract was diluted by using a cell culture medium, as known as complete medium (a medium that added FBS 10%, penicillin/ streptomycin 1%, sodium pyruvate 1%, non-essential amino acid 1%, HEPES 2.5% and β- mercaptoethanol 0.1% to Gibco RPMI). The same amount of DMSO, a solvent, and extract were added to a complete medium and the resulting solution was used as the control group.

*Preparation of cells*

After putting the mouse to euthanasia with $CO_2$, spleens and lymph nodes were separated and passed through a 70 μm strainer, which led to the acquisition of unicellular, suspended matters. In order to obtain naive CD4 T cells, it was dyed with CD25-PE antibodies (eBioscience) and attached with anti-PE, anti-CD8α, anti-B220 and anti-CD11b bead (Miltenyl Biotec). Then, it underwent depletion using MACS (Magnetic-Activated Cell Sorting), resulting in an enrichment of CD4 T cells. Afterwards, CD62L-biotin antibodies (eBioscience) and anti-biotin bead (Miltenyl Biotec) were attached and naive CD4 T cells were gathered through MACS. The unicellular suspended matters obtained from spleens and lymph nodes were dissolved in RBC and attached with CD3-biotin antibodies (eBioscience) and anti-biotin bead (Miltenyl Biotec) before undergoing MACS depletion. The resulting cells were used as antigen-presenting cells.

*In vitro cell culture*

All cell culture was performed in a complete medium. In the case of antibody stimulation, 2 $\mu$g/ml of anti-CD3 was coated over 96 well flat plates throughout one night, and was added with dissolution of $5\times10^4$ cells/well of naive CD4 T cells along with 1 $\mu$g/ml of anti-CD28. In the case of stimulating antigen-presenting cells, the dissolved anti-CD3 antibodies were added to the 96 well U-bottom plates, and $6\times10^4$ cells/well of antigen-presenting cells were cultivated with $3\times104$ cells/well of naive CD4 T cells. In both cases, a recombined mouse IL-2 was added with the concentration of 10 ng/ml, and the extract diluted to a desired concentration was then added to each well. These were cultivated under the conditions of 37 °C and 5% $CO_2$ for 72 hours before the cells were collected and analyzed.

*Foxp3 dyeing in cells and flow cytometric analysis*

The cultivated cells were withdrawn and dyed on the surface for 15 minutes by using a buffer solution, a mixture of PBSN (0.14M NaCl, 0.003M KCl, 0.01M $Na_2HPO_4$, 0.002M $KHPO_4$, 0.002M $NaN_3$) and 1% FBS, and CD4-PE/Cy7 (Biolegend). Afterwards, eBioscience's Foxp3 dye set was used to stain Foxp3 in cells, according to specified instructions. For the FACS analysis, BD FACS Calibur and Cell Questpro were used.

An investigation of the effects that *Poria cocos* Wolf bark extract, *Cynanchym atratum* Bunge extract or *Arisaema amurense* extract have on Foxp3 expression in cells showed that the Foxp3 expression had more than doubled by 10 $\mu$g/ml of the *Poria cocos* Wolf bark extract or *Cynanchym atratum* Bunge extract, compared to the control group. If *Arisaema amurense* extract is processed, the Foxp3 expression showed concentration-dependent increase.

These results represent that *Poria cocos* Wolf bark extract, *Cynanchym atratum* Bunge extract or *Arisaema amurense* extract induces differentiation into Treg cells (fig. 4a, 5a and 6a).

*Cytokine analysis*

The cytokine production effect in spleen of *Poria cocos* Wolf bark extract, which are revealed as cytokines of supernatant of cultured spleen cell were analyzed like the method of Example 3, were examined. If 10 $\mu$g/ml of *Poria cocos* Wolf bark extract is added, production of TGF-$\beta$ which is Treg cytokine increased by 70.3%, compared to a control group (the bottom of the fig. 4b). The effect of increased TGF-$\beta$ production confirm that Poria cocos Wolf bark extract induces differentiation into Treg cells.

*Statistical analysis*

Student's t-test is used for significance evaluation of the results.

**Example 6: Stabilizing activity of Treg cells**

Many of components act on foxp3 expression in Treg cells. The expression activity of foxp3 gene promoter in itself is weak, so other foxp3 regulatory components have an effect on chromatins and are involved in the expression. For example, in foxp3-CNS1 (Forkhead box P3-Conserved Noncoding Sequence1), there are binding sites against Smad-3 which is activate by NFAT (Nuclear Factor of Activated T-cells) and TGF-$\beta$ (Transforming Growth Factor-beta), it plays an important role in induction of naive CD4 T cells into Treg cells in periphery.

The foxp3 expression is not maintained permanently, usually disappeared by many factor such as changes of methylation. According to a published research, when OX40(CD134), which is a member of TNF (Tumor Necrosis Factor) receptor superfamily, is cross-linked, the foxp3 expression and immunosuppressive activity of Treg cell are disappeared. Therefore, Treg cell stabilizing activity of *Poria cocos* Wolf bark extract, *Cynanchym atratum* Bunge extract and *Arisaema amurense* extract was verified by maintenance of foxp3 expression.

The foxp3-GFP mice are used in order to verify maintenance of foxp3 expression by *Poria cocos* Wolf bark extract, *Cynanchym atratum* Bunge extract and *Arisaema amurense* extract. Because the foxp3-GFP mice express foxp3 and GFP fluorescence in nucleus, it is possible to identify without nucleus staining process. Because the mice is C57BL/6 line, the effects of foxp3 induction in Balb/c mice were confirmed in C57BL/6 mice, and there are the same effects in C57BL/6 mice. The natural Treg cells generated thymus of Foxp3-GFP mice express fluorescence in itself, so the Treg cells could be separated by FACS and we confirmed that the purity of the cells is near 100%.

When the Treg cells were cultured for 3 days, a ratio of foxp3 expressing cells was decreased by 63.43% (ctrl: a control group). This happens because foxp3 expression is regulated by many components, and the foxp3 maintenance conditions are not satisfied as mentioned above.

On the other hand, if 10 $\mu$g/ml of *Poria cocos* Wolf bark extract, 10 $\mu$g/ml of *Cynanchym atratum* Bunge or 100 $\mu$g/ml of *Arisaema amurense* extract is processed, the ratio of foxp3 expression was maintained high, compared to a control group. In other words, the ratio of foxp3 expression was maintained by 74.91% in case of treatment of *Poria cocos* Wolf bark extract, 75.78% in case of treatment of *Cynanchym atratum* Bunge extract, and 69.98% in case of treatment of *Arisaema amurense* extract, and the ratio of foxp3 expression is higher than that of the control group, therefore, we found that *Poria cocos* Wolf bark extract, *Cynanchym atratum* Bunge extract or *Arisaema amurense* extract has stabilizing activity of Treg cells (fig. 4b, 5b and 6b).

**Example 7: Test for in vivo anti-allergic activity of Poria cocos Wolf bark extract**

5-week-old female BALB/c mice were purchased and OVA (20 $\mu$g) and alum (2 mg) were intraperitoneally injected twice to the mice for immune response after a week of adjustment. 25 mg/kg of 95% EtOH *Poria cocos* Wolf bark extract was orally administered every day for 10 days on each mouse, and blood was gathered and spleen and mesenteric lymph node were separated after the final oral administration.

The serum was separated from the blood, and used for analysis of IgE, IgG2a and IgG1. The spleen and the mesenteric lymph node were monocellularized and were processed with 100 $\mu$g/ml of OVA. These were cultivated for 72 hours and cytokines in culture supernatant were analyzed. ELISA kits (BD bioscience) used to analyze Cytokines including IL-5, IL-10 and IL-13 as Th2-related cytokines, INF-$\gamma$ as Th1-related cytokines, and TGF-$\beta$ as Treg cytokines.

If 95% EtOH *Poria cocos* Wolf bark extract decrease IgE, IgG2a and IgG1 by about 37.9%, 38.4% and 49.7%, respectively, compared to a control group (fig. 7a), IL-5, IL-10 and IL-13 by about 43.1%, 56.8% and 17.3% (fig. 7b), INF-$\gamma$ by about 38.8%(fig. 7c), and increase TGF-$\beta$ by about 37% (fig. 7d).

Therefore, *Poria cocos* Wolf bark extract has anti-allergenic activity because the extract decrease IgE, IgG2a and IgG1 of serum in vivo, Th2- and Th1-related cytokines, and increase Treg cytokines.

**Example** 8: **Test for activity of *Poria cocos* Wolf bark extract in food allergy models.**

*Preparation of food allergy models*

5-week-old female BALB/c mice were purchased and OVA (20 $\mu$g) and alum (2 mg) were intraperitoneally injected to the mice after a week of adjustment in order to induce allergies. 25 mg/kg (body weigh) of *Poria cocos* Wolf bark extract was orally administered every day, and 50 mg of OVA was orally administered together every 3 days in order to induce food allergies. For positive control groups, Dexamethason (2.5 mg/kg (body weight)) were administered like the *Poria cocos* Wolf bark extract administered group. Fig. 8 represents the specific schedules.

*Measurements of diarrhea score, anaphylatic response and change of rectal temperature*

Diarrhea score, anaphylatic response and change of rectal temperature as a food allergenic indicator were observed every 15 minutes for 60 minutes when the oral administration of the 4th, 5th and 6th treatment of *Poria cocos* Wolf bark extract with OVA. The *Poria cocos* Wolf bark extract reduced diarrhea score, anaphylatic response and change of rectal temperature, compared to a control group (fig. 9). Therefore, *Poria cocos* Wolf bark extract reduce induction of food allergy.

*Analysis of cytokines in cellularized spleen supernatant*

The spleens of food allergy models were monocellularized and processed with OVA 100 $\mu$g/ml. These were cultivated for 72 hours and the culture supernatant was collected and cytokines of which were analyzed. The analysis was performed by using an BD OptEIATM MOUSE ELISA(Enzyme-linked Immunosorbent Assay) according to the protocol of the manufacturer. The *Poria cocos* Wolf bark extract reduced the productions of IL-4, IL-5, IL-10 and IL-13 as Th2-related cytokines of spleen cells by about 47.3%, 34.5%, 36.7% and 37.7%, respectively (fig. 10a and 10b), increase TGF-$\beta$ as Treg cytokine which is related in induction of foxp3 expression *in vivo* by about 51.6% (fig. 10c).

The effects of decrease in production of Th2-related cytokines and increase in production of foxp3 expression induction cytokines by *Poria cocos* Wolf bark extract represent induction of differentiation into Treg cells.

**Example 9: Activating fraction discovery of *Poria cocos* Wolf bark extract**

*Separation of solvent fraction*

The freeze dried 95% EtOH extract Of *Poria cocos* Wolf bark was melted in water and first extracted by hexane which is low-polarity solvent. After filterating, the samples were sequentially separated 3-4 times by using chloroform, ethyl acetate and butanol, and separated into five fractions. We named the five fractions Hexane Fr., Chloroform Fr.,

Ethyl acetate Fr., Butanol Fr. And Water Fr. (fig. 11a).

*Activating fraction of Poria cocos Wolf bark extract*

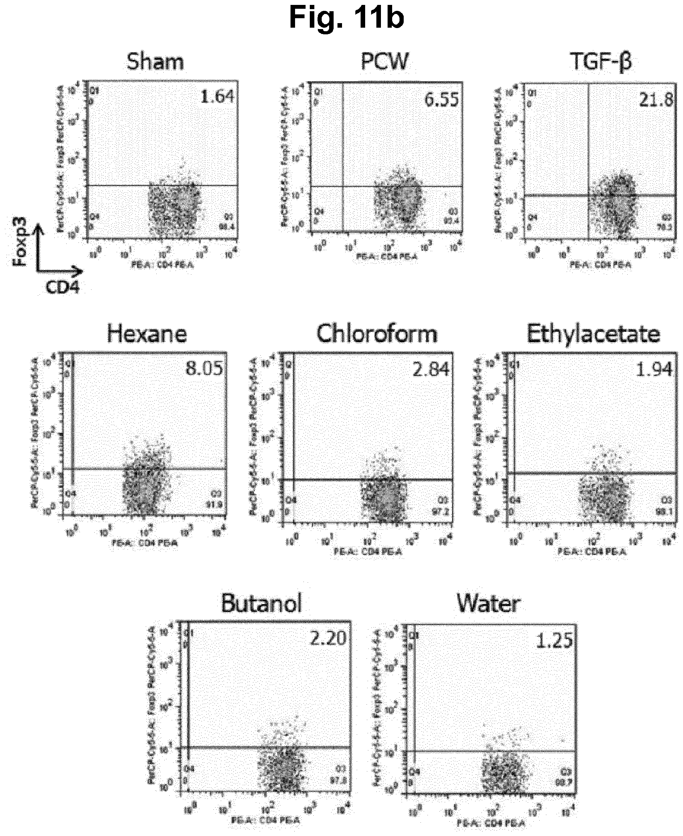

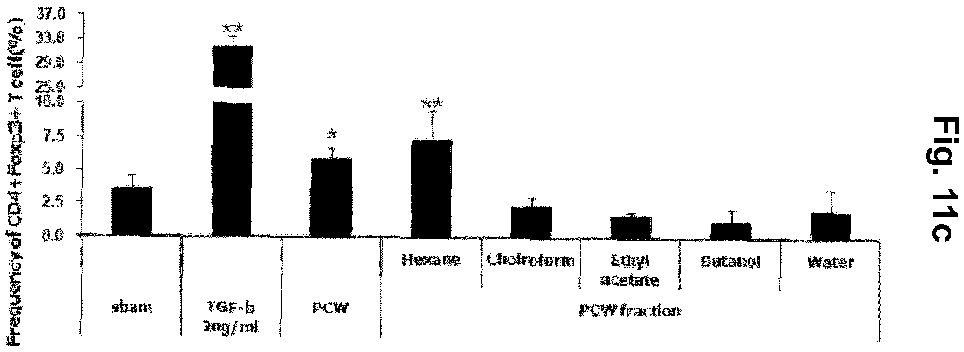

The foxp3 induction activities of the five fractions were measured by the above mentioned methods in Example 5. We found that the hexane fraction had foxp3 induction activities (fig. 11b and 11c).

**Claims**

1. A composition comprising *Poria cocos* Wolf bark extract as active ingredient for use in preventing, improving, or treating Th2-mediated allergic diseases.

2. Use of a composition *in vitro* comprising *Poria cocos* Wolf bark extract for inducing differentiation of splenocytes and lymphatic gland cells from a subject into Treg cells.

3. Use of a composition *in vitro* comprising *Poria cocos* Wolf bark extract for increasing the production of TGF-β (Transforming Growth Factor-beta).

4. Use of a composition *in vitro* comprising *Poria cocos* Wolf bark extract for stabilizing the activity of Treg cells as verified by the maintenance of the foxp3 gene expression.

5. Use of a composition *in vitro* comprising *Poria cocos* Wolf bark extract for inhibiting the degranulation of mast cells.

6. Use of a composition *in vitro* comprising *Poria cocos* Wolf bark extract for inhibiting the production of one or more Th2-related cytokines selected from the group consisting of IL-4, IL-5, IL-10 and IL-13.

7. Use of a composition *in vitro* comprising *Poria cocos* Wolf bark extract for inhibiting the production of one or more Th1-related cytokines selected from the group consisting of IL-17 and IFN-γ.

8. Use of a composition *in vitro* comprising *Poria cocos* Wolf bark extract for inhibiting the production of one or more antibodies selected from the group consisting of IgE, IgG2a and IgG1 in serum.

9. A composition for use according to claim 1, comprising *Poria cocos* Wolf bark extract as active ingredient, wherein the composition is a pharmaceutical composition, food composition, functional food composition, or a fodder composition.

**Patentansprüche**

1. Zusammensetzung, umfassend Rindenextrakt von *Poria cocos* Wolf als Wirkstoff zur Verwendung bei Vorbeugen, Verbessern oder Behandeln von Th2-vermittelten allergischen Erkrankungen.

2. Verwendung einer Zusammensetzung *in vitro,* umfassend Rindenextrakt von *Poria cocos* Wolf zum Induzieren von Differenzierung von Splenozyten und Lymphdrüsenzellen von einem Subjekt in Treg-Zellen.

3. Verwendung einer Zusammensetzung *in vitro,* umfassend Rindenextrakt von *Poria cocos* Wolf zum Erhöhen der Produktion von TGF-β (Transforming Growth Factor-beta).

4. Verwendung einer Zusammensetzung *in vitro,* umfassend Rindenextrakt von *Poria cocos* Wolf zum Stabilisieren der Aktivität von Treg-Zellen, wie durch das Aufrechterhalten der foxp3-Genexpression verifiziert.

5. Verwendung einer Zusammensetzung *in vitro,* umfassend Rindenextrakt von *Poria cocos* Wolf zum Hemmen der Degranulation von Mastzellen.

6. Verwendung einer Zusammensetzung *in vitro,* umfassend Rindenextrakt von *Poria cocos* Wolf zum Hemmen der Produktion von einem oder mehreren Th2-bezogenen Zytokinen, ausgewählt aus der Gruppe bestehend aus IL-4, IL-5, IL-10 und IL-13.

7. Verwendung einer Zusammensetzung *in vitro,* umfassend Rindenextrakt von *Poria cocos* Wolf zum Hemmen der Produktion von einem oder mehreren Th1-bezogenen Zytokinen, ausgewählt aus der Gruppe bestehend aus IL-17 und IFN-γ.

8. Verwendung einer Zusammensetzung *in vitro,* umfassend Rindenextrakt von *Poria cocos* Wolf zum Hemmen der Produktion von einem oder mehreren Antikörpern, ausgewählt aus der Gruppe bestehend aus IgE, IgG2a und IgG1 in Serum.

9. Zusammensetzung zur Verwendung nach Anspruch 1, umfassend Rindenextrakt von *Poria cocos* Wolf als Wirkstoff, wobei die Zusammensetzung eine pharmazeutische Zusammensetzung, eine Nahrungsmittelzusammensetzung, eine funktionelle Nahrungsmittelzusammensetzung oder eine Futtermittelzusammensetzung ist.

## Revendications

1. Composition comprenant un extrait d'écorce de *Poria cocos* Wolf comme ingrédient actif pour utilisation dans la prévention, l'amélioration ou le traitement de maladies allergiques à médiation par Th2.

2. Utilisation d'une composition *in vitro* comprenant un extrait d'écorce de *Poria cocos* Wolf pour induire une différenciation des splénocytes et des cellules des glandes lymphatiques d'un sujet dans des cellules Treg.

3. Utilisation d'une composition *in vitro* comprenant un extrait d'écorce de *Poria cocos* Wolf pour augmenter la production de TGF-β (facteur de croissance transformant bêta).

4. Utilisation d'une composition *in vitro* comprenant un extrait d'écorce de *Poria cocos* Wolf pour stabiliser l'activité de cellules Treg comme vérifiée par le maintien de l'expression du gène foxp3.

5. Utilisation d'une composition *in vitro* comprenant un extrait d'écorce de *Poria cocos* Wolf pour inhiber la dégranulation de mastocytes.

6. Utilisation d'une composition *in vitro* comprenant un extrait d'écorce de *Poria cocos* Wolf pour inhiber la production d'une ou plusieurs cytokines associées à Th2 sélectionnées parmi le groupe constitué de IL-4, IL-5, IL-10 et IL-13.

7. Utilisation d'une composition *in vitro* comprenant un extrait d'écorce de *Poria cocos* Wolf pour inhiber la production d'une ou plusieurs cytokines associées à Th1 sélectionnées parmi le groupe constitué de IL-17 et IFN-γ.

8. Utilisation d'une composition *in vitro* comprenant un extrait d'écorce de *Poria cocos* Wolf pour inhiber la production d'un ou plusieurs anticorps sélectionnés parmi le groupe constitué de IgE, IgG2a et IgG1 dans le sérum.

9. Composition pour utilisation selon la revendication 1, comprenant un extrait d'écorce de *Poria cocos* Wolf comme principe actif, dans laquelle la composition est une composition pharmaceutique, une composition alimentaire, une composition alimentaire fonctionnelle ou une composition de fourrage.

# Fig. 1a

# Fig. 1b

# Fig. 1c

# Fig. 2a

# Fig. 2b

# Fig. 2c

# Fig. 3a

*Poria cocos* Wolf bark

# Fig. 3b

# Fig. 3c

EP 2 929 888 B1

# Fig. 4a

(Anti-CD3/28)

(Anti-CD3/APC)

26

# Fig. 4b

# Fig. 4c

**control**

**Poria cocos Wolf bark**

# Fig. 5a

# Fig. 5b

control

*Cynanchym atratum* Bunge

# Fig. 6a

# Fig. 6b

# Fig. 7a

# Fig. 7b

# Fig. 7c

# Fig. 7d

# Fig. 8

# Fig. 9a

# Fig. 9b

# Fig. 9c

# Fig. 10a

# Fig. 10b

# Fig. 10c

# Fig. 11a

**95% Ethanol extracts**

Extracted with 70% ethanol
evaporated in vacuum

Ethanol extract

Hexane : **95%** ethanol extracts : $H_2O$ (10 : 1 : 9)

Hexane layer          Water layer

Extraction with Chloroform

Evaporation

**Hexane Fr.**  Aqueous layer         Chloroform layer

Extraction with Ethyl acetate

Evaporation

Ethyl acetate layer      Water layer  **Chloroform Fr.**

Extraction with butanol

Evaporation

**Ethyl acetate Fr.**  Water layer         Butanol layer

Evaporation               Evaporation

**Water Fr.**              **Butanol Fr.**

# Fig. 11b

# Fig. 11c

**EP 2 929 888 B1**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 1498130 A1

- EP 2305268 A1

**Non-patent literature cited in the description**

- **JUN HO LEE et al.** Rubiae Radix suppresses the activation of mast cells through the inhibitions of Syk kinase for anti-allergic acrivity. *journal of pharmacy and pharmacology,* 01 April 2006, vol. 58 (4), 503-512

- Remington's Pharmaceutical Sciences. 1995